(19) Europäisches Patentamt / European Patent Office / Office européen des brevets

(11) **EP 4 255 297 B1**

(12) **EUROPEAN PATENT SPECIFICATION**

(45) Date of publication and mention of the grant of the patent:
**20.08.2025 Bulletin 2025/34**

(21) Application number: **21851875.1**

(22) Date of filing: **06.12.2021**

(51) International Patent Classification (IPC):
*A61B 5/12* (2006.01)    *A61B 5/00* (2006.01)
*G16H 50/30* (2018.01)

(52) Cooperative Patent Classification (CPC):
**A61B 5/125; A61B 5/7275; G16H 50/30**

(86) International application number:
**PCT/US2021/062073**

(87) International publication number:
**WO 2022/125472 (16.06.2022 Gazette 2022/24)**

(54) **METHODS AND SYSTEMS FOR AUDIOMETRIC EARLY DETECTION, PREDICTION AND AGGREGATE TREND ANALYSIS OF NOISE-INDUCED AND OTHER PROGRESSIVE HEARING LOSS**

VERFAHREN UND SYSTEME ZUR AUDIOMETRISCHEN FRÜHERKENNUNG, VORHERSAGE UND AGGREGATTRENDANALYSE VON RAUSCHINDUZIERTEM UND ANDEREM PROGRESSIVEM HÖRVERLUST

MÉTHODES ET SYSTÈMES DE DÉTECTION PRÉCOCE AUDIOMÉTRIQUE, DE PRÉDICTION ET D'ANALYSE DES TENDANCES GLOBALES D'UNE PERTE AUDITIVE INDUITE PAR LE BRUIT ET D'AUTRES PERTES AUDITIVES PROGRESSIVES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **07.12.2020 US 202063122083 P**

(43) Date of publication of application:
**11.10.2023 Bulletin 2023/41**

(73) Proprietors:
• **Craner, James**
  **Reno, Nevada 89511 (US)**
• **Willits, Neil**
  **Davis, California 95616 (US)**

(72) Inventors:
• **Craner, James**
  **Reno, Nevada 89511 (US)**
• **Willits, Neil**
  **Davis, California 95616 (US)**

(74) Representative: **Mewburn Ellis LLP**
  **Aurora Building**
  **Counterslip**
  **Bristol BS1 6BX (GB)**

(56) References cited:
  **US-A1- 2017 300 631**

• **HONG OISAENG ET AL: "Understanding and preventing noise-induced hearing loss", DISEASE-A-MONTH, ELSEVIER, AMSTERDAM, NL, vol. 59, no. 4, 16 March 2013 (2013-03-16), pages 110 - 118, XP029005478, ISSN: 0011-5029, DOI: 10.1016/J.DISAMONTH.2013.01.002**
• **HOUSER DORIAN S ET AL: "A review of the history, development and application of auditory weighting functions in humans and marine mammals", THE JOURNAL OF THE ACOUSTICAL SOCIETY OF AMERICA, AMERICAN INSTITUTE OF PHYSICS, 2 HUNTINGTON QUADRANGLE, MELVILLE, NY 11747, vol. 141, no. 3, 2 March 2017 (2017-03-02), pages 1371 - 1413, XP012216703, ISSN: 0001-4966, [retrieved on 20170302], DOI: 10.1121/1.4976086**

(Cont. next page)

• JANSEN E J M ET AL: "Noise induced hearing loss and other hearing complaints among musicians of symphony orchestras", INTERNATIONAL ARCHIVES OF OCCUPATIONAL AND ENVIRONMENTAL HEALTH, SPRINGER, BERLIN, DE, vol. 82, no. 2, 11 April 2008 (2008-04-11), pages 153 - 164, XP019714895, ISSN: 1432-1246

• DEMEESTER K ET AL: "Heritability of audiometric shape parameters and familial aggregation of presbycusis in an elderly Flemish population", HEARING RESEARCH, ELSEVIER SCIENCE PUBLISHERS , AMSTERDAM, NL, vol. 265, no. 1-2, 14 June 2010 (2010-06-14), pages 1 - 10, XP027051012, ISSN: 0378-5955, [retrieved on 20100318]

**Description**

CROSS-REFERENCES TO RELATED APPLICATIONS

This claims the benefit of the filing date of U.S. Provisional Application No. 63/122,083, filed December 7, 2020.

FIELD OF THE INVENTION

The invention relates to occupational safety and health as it pertains to noise exposure and hearing. In particular, aspects of the invention provide methods to mathematically transform individual audiometric test data into a single numerical metric that summarizes the magnitude of hearing loss specifically toward early noise-induced hearing loss.

BACKGROUND OF THE INVENTION

US 2017/300631 shows a method to estimate real ear exposure levels and predict future hearing loss parameters. Noise-induced hearing loss ("NIHL") represents one of the most prevalent and costly occupational diseases in the industrialized world. It is the most common occupational disease (illness) reported in the United States. NIHL affects all demographics and disproportionately impairs younger workers.

Work-related NIHL is a largely, if not completely, preventable disease. NIHL develops in response to cumulative exposure to excessive levels of noise in a wide variety of industries and occupations. The disease has a relatively rapid progression from onset of exposure to the time of detection, a point at which NIHL is irreversible. Past and current methods of preventing the disease have consisted of regulations limiting workers' exposure to noise and requiring them to wear personal protective equipment ("PPE").

Occupational noise regulations include, without limitation, the United States Occupational Safety and Health Administration ("OSHA") 29 CFR 1910.95 and the Mine Safety and Health Administration ("MSHA") 30 CFR Part 62 (collectively the "Noise Standards"), and industry guidelines and comparable regulations in other nations (e.g., Canadian Standards Association Standard Z107.6-16 "Audiometric Testing for Use in Hearing Loss Prevention Programs" and Z1007-16 "Hearing Loss Prevention Program (HLPP) Management"). These specification-based regulations and guidelines require primary preventive measures consisting of engineering controls, work practice control measures, and use of PPE, namely hearing protective devices ("HPDs") such as ear plugs and ear muffs, to reduce workers' noise exposure dose below the permissible exposure limit ("PEL") of 90 decibels (dB). The decibel is a unit of measure of the intensity of a sound or the power level of an electrical signal by comparing it with a given level on a logarithmic scale.

The above-referenced regulations require the employer to institute a hearing conservation program ("HCP") if an 8-hour time-weighted average sound level exposure equals or exceeds the "action level," which under OSHA and MSHA is 85 decibels (dB). The purpose of the HCP as a form of secondary prevention is to accurately identify early NIHL and prevent it from becoming irreversible and advanced in workers occupationally exposed to noise. Employees in a HCP undergo training and audiometric testing ("audiogram") before starting work in a noise-exposed job (to establish a hearing "baseline") and periodically (typically annually) thereafter.

For screening audiograms conducted as part of HCPs, hearing levels (also commonly referred to as "thresholds") at each sound frequency are recorded separately for each ear in decibels in interval (non-continuous) increments of 5 dB. At its earliest stages, NIHL impacts hearing thresholds (i.e., causes a decline in hearing) in the higher sound frequencies (4 kHz and 6 kHz, occasionally 3 kHz in selected industries or exposures). These frequencies are mostly outside the human speech range, and thus a subject's diminished ability to hear them does not impair regular hearing or otherwise cause symptoms.

NIHL has a characteristic, diagnostic audiometric pattern of a high frequency hearing loss "notch" with a peak threshold (also referred to as a hearing "loss") at 4 kHz or 6 kHz (and occasionally at 3 kHz) with recovery (i.e., return toward 0 dB) at 8 kHz. As NIHL progresses in an individual with continued exposure to excessive noise, it impairs the ability to hear lower, speech-range sound frequencies (0.5, 1 and 2 kHz), and thus impacts speech perception. Beyond this point, with further advancement of NIHL, the audiogram shape typically deforms and loses its characteristic, diagnostic "notch" appearance. Advanced NIHL results in a variable amount of irreversible hearing loss, sometimes accompanied by tinnitus (ringing of the ears), that can cause permanent partial disability, significantly impair social life and work, and require the use of a hearing aid.

Clinical interpretation of individual audiograms is a complex process. For interpretation of serial screening audiograms, an objective, generally accepted or validated criterion (or set of criteria) does not exist to detect and measure the earliest audiometric signs of occupational NIHL, or to recognize and differentiate them from any other disease or condition that produces hearing loss. In practice, when a screening audiogram is interpreted or compared to a baseline or previous audiograms, it is typically "eye-balled," i.e., reviewed without being subjected to any formal statistically analysis, for each employee, one-at-a-time, and then filed away for recordkeeping compliance purposes unless specific administrative action is required.

The Noise Standards do not even require a medical or hearing professional to clinically interpret audiograms, either individually or in aggregate, nor do they specify a particular method or criterion for their interpretation. Aside from the computation of a non-specific Standard Threshold Shift (as described below), neither the regulations nor any professional standards require or provide guidelines for statistical analysis of individual or aggregate trends among similar exposure groups ("SEG"s) of workers in audiometric hearing loss progression over time relative to measured or estimated workplace noise exposures.

Uncertainties in clinical sensitivity and specificity are a major limitation in the reliability and effectiveness of serial screening audiograms as a secondary preventive method for occupational NIHL. Studies have demonstrated that even when trained audiologists, otolaryngologists and occupational medicine physicians review an individual's screening audiogram results and test-to-test changes, their interpretation methodology is subjective and their interpretations vary widely in consistency. This inconsistency arises because the earliest audiometric abnormalities indicative of NIHL are difficult to distinguish from what constitutes a "normal" audiogram. The pattern and timing of audiometric progression from normal to abnormal for NIHL has many different presentations which are time- and exposure-dependent. No specific numerical criterion defines where audiometric normalcy ends and NIHL disease begins. Most audiologists and other hearing professionals consider hearing loss thresholds of ≤15 dB or ≤20 dB at any frequency as within the normal range for adults, and many organizations and regulatory agencies do not consider hearing to be "abnormal" until at least one frequency is ≥20 or 25 dB. A universally accepted absolute or relative (e.g., percent) criterion for a significant year-to-year (test-to-test) change does not exist. Intra-person test-to-test variability (+/- 5 dB at any frequency) further impacts the ability to reliably distinguish a true positive test result (one indicating clinically relevant hearing loss) from a false positive test result (one with non-clinically relevant hearing loss with one or more elevated hearing levels reflecting normal variability).

As a result, an individual subject's serial audiograms may have small, seemingly minor or fluctuating year-to-year changes that represent the earliest stage of reversible NIHL disease which go unnoticed, whereas only when a large overall change from baseline occurs in a recognizable pattern may an irreversible noise-induced hearing loss in either or both ears be reliably detected and diagnosed.

Interpretation of an individual worker's series of screening audiograms is further complicated by the inherently variable natural history of NIHL disease progression which is dependent on individual health, work conditions, cumulative and peak noise levels, and work practices, including the use of HPDs. All individuals exposed to certain levels, types and duration of noise are susceptible to NIHL, but only some of those who have been adequately exposed will develop the disease. The onset of NIHL typically begins after 4-10 years of sufficiently high noise exposure and can occur even with regular use of HPDs. Non-occupational sources of noise exposure and certain medical conditions can contribute to hearing loss progression, or confound the audiometric diagnosis of early NIHL. Some employees may have an abnormal baseline audiogram reflecting pre-existing NIHL or other forms of hearing loss. Even when noise exposure affects both ears equally, the progression of audiometric changes may not be symmetrical.

Hearing tends to either stay the same or worsen in adults over time. Only in a few diseases or conditions (e.g., cerumen removal, or treatment of middle ear infection) does hearing improve significantly. In persons over 50 years of age, NIHL in its advanced stages can be difficult to audiometrically and clinically differentiate from presbycusis, a common, age-related cause of high frequency hearing loss. Numerous other variables related to noise exposure, employee, physician, and disease course also complicate the accurate diagnosis and causal assessment of NIHL on an individual basis.

As mentioned above, the Threshold Shift, i.e., STS ("Standard Threshold Shift" in the United States, and "Significant Threshold Shift" in Canada) is the conventional, regulatorydefined metric for occupational audiograms deemed to indicate irreversible hearing loss. Either or both ears can sustain a STS, simultaneously or separately, and a single individual can incur multiple STS occurrences over time. In the United States, OSHA and MSHA define a STS ("STS-OSHA," in either ear) as the decline (as compared with baseline) of the arithmetic average of hearing threshold at 2, 3 and 4 kHz equal to or greater than 10 dB, as shown in the calculation below:

$$(L_{2kHzCurrent} + L_{3kHzCurrent} + L_{4kHzCurrent})/3 - (L_{2kHzBaseline} + L_{3kHzBaseline} + L_{4kHzBaseline})/3 \geq 10 \text{ dB}$$

where L = threshold of detection of the sound at the given frequency, in dB.
An OSHA *recordable* STS has the added criterion:

$$(L_{2kHzCurrent} + L_{3kHzCurrent} + L_{4kHzCurrent})/3 \geq 25 \text{ dB}.$$

Thus, a "recordable" STS ("STS-REC-OSHA") requires at least one of the 2, 3 or 4 kHz hearing levels to be ≥25 dB. This criterion was added by OSHA to prevent false positives that are within the normal range of hearing. Whether or not such non-recordable STSs predict or are a sign of subsequent early noise-induced hearing loss is unknown.

Under the Canadian Standards Association Z107.6-16 (2016), a Significant Threshold Shift ("STS-CSA"), in either ear) is defined as:

$$[(L_{2kHzCurrent} + L_{3kHzCurrent} + L_{4kHzCurrent})/3 - (L_{2kHzBaseline} + L_{3kHzBaseline} + L_{4kHzBaseline})/3 \geq 10$$

$$dB \ AND \ (L_{2kHzCurrent} + L_{3kHzCurrent} + L_{4kHzCurrent})/3 \geq 30 \ dB]$$

$$OR \ (L_{3kHzCurrent} - L_{3kHzBaseline}) \geq 15 \ dB$$

$$OR \ (L_{4kHzCurrent} - L_{4kHzBaseline}) \geq 15 \ dB.$$

Though the Noise Standards consider the STS to be an "early indicator of permanent hearing loss," no scientific evidence has been published to validate the STS-OSHA (or STS-CSA) as an effective preventive metric. At the time the OSHA Noise Standard was instituted in the early 1980s, the regulatory STS-OSHA definition and its criterion ("cutoff") value of $\geq$ 10 dB was promulgated by OSHA through consensus, and not through published scientific studies. Since that time (nearly 40 years), no scientific research has been conducted to validate or challenge this criterion.

The STS (for every Standard) is intended for individual worker determination, but not for measuring aggregate (SEG) trends toward or past the point of early NIHL. Thus, a STS in and of itself does not clearly demarcate a significant change in *reversible* NIHL, either individually or among SEGs or the entire population of employees in the HCP. Often, by the time a STS is detected for an individual, it is too late to prevent or reverse the disease process. Thus, the STS is a non-specific, lagging indicator of (irreversible) disease that relies upon and is limited to one-test (and one-ear)-at-a-time determinations, with or without subjective interpretations of the entire individual audiogram.

In conjunction with the limitations discussed above with regard to audiometric interpretation, the audiometric test performance characteristics-sensitivity and specificity-of the STS to detect of the earliest phase of NIHL have never been systematically analyzed or determined. Sensitivity is the probability an abnormal ("positive") test correctly identifies the *presence* of the disease. The STS is problematic with regard to sensitivity because NIHL characteristically starts in the high frequency hearing range, but in many cases by the time it impacts the 2 kHz (speech) range-which the STS includes-hearing loss is already moderately advanced and irreversible. The 6 kHz threshold, in contrast, is much more sensitive to early NIHL changes, but it is *not* included in the STS definition. Specificity is the probability a normal ("negative") test correctly identifies the *absence* of disease. The specificity of the STS is similarly inadequate because when NIHL advances to the point where a STS has occurred, the pattern may not be accurately differentiated from common diseases such as presbycusis or other less prevalent diseases associated with high frequency hearing loss. This common situation creates a false positive STS which must nonetheless be reported and medically evaluated. Further, the Noise Standards contain outdated age-adjustment formulas that unreliably filter out the effect of older age on STS values, thereby misattributing some noise-related hearing loss to age-related hearing loss (presbycusis) in older workers. Consequently, contestation of such cases for purposes of occupational injury recordability and workers' compensation claim adjudication consumes a substantial amount of resources that contribute to the large economic burden of NIHL.

While individual clinical diagnosis and screening for NIHL through audiograms is an important component to occupational disease control, a public health (i.e., population-based) approach represents the most effective method to reduce occupational disease risk through intervention. The need for and importance of quantitatively evaluating HCP effectiveness within a company by utilizing audiometric data was first recognized in the late 1980s. The term "audiometric database analysis" ("ADBA") was proposed to describe a standardized, systematic method of aggregate statistical analysis of serial audiograms in individual employees. ADBA is described more fully in Hearing Conservation Programs: Practical Guidelines for Success, Julia Royster and Larry H. Royster © 1990. The purpose of ADBA is (1) early identification and measurement of aggregate trends among similarly exposed workers in a given workplace that would prevent threshold shifts, and (2) objective statistical evaluation of the overall effectiveness of the HCP among each group of workers, across departments, or for the entire facility.

After more than 30 years of professional efforts to develop a robust ADBA methodology for this purpose, no consensus-based criteria (e.g., the American National Standards Institute ("ANSI") S12.13 Standard) or mathematical modeling methods have ever been scientifically validated or widely adopted. Even with the widespread use of computerization and availability of databases, internet, and automated information technology, no substantive methodological or technological advances have been developed or widely implemented for employers or other stakeholders to utilize audiometric data to statistically analyze aggregate audiometric trends over time to objectively measure risk for NIHL. Neither governmental agencies, nor any professional (medical, audiological, or industrial hygiene) organizations, nor any audiometry hardware or software manufacturers or distributors have developed or offer any audiometric analytical methods or tools to objectively measure HCP effectiveness. Thus, the *de facto* "standard of care" for HCP audiometric data remains limited to fulfilling the minimum recordkeeping and one-test-at-a-time audiometric STS requirements mandated by the OSHA and MSHA Noise Standards, or their international counterparts.

While billions of dollars have been spent over the past 40 years satisfying regulatory compliance requirements for noise, the incidence of NIHL continues unabated, making it one of the most prevalent occupational diseases. The magnitude and extent to which HCPs within companies or industries are effectively controlling the risk of NIHL within their worker populations remains largely unknown. The reasons why audiometric data remains largely unactionable include the aforementioned inherent complexity of the audiogram, reliance upon one-test-at-a-time expert interpretation, the STS metric as a crude lagging indicator of early disease, and the lack of an adequate ADBA methodology and system.

To make audiometric data actionable, there is a need for a method of calculating a metric that accurately *summarizes* individual audiometric results (data) for hearing loss specifically toward NIHL. To such a summary metric, *post hoc* statistical methods can be applied to analyze audiometric data trends within and among individuals, SEGs or any designated population within a company or organization. Widespread implementation of such population-based, statistical analytical approaches can transform compliance-driven, individual screening testing with limited preventive capability into medical surveillance processes that can be directly linked to corrective and prevention actions for individuals and groups of workers, as more fully set forth in Craner, J. "Medical Surveillance" (chapter 41) in Current Occupational and Environmental Medicine, Fifth Edition, LaDou J and Harrison R (Eds.), McGraw Hill Companies, Inc., 2014.

There is a further need for a computerized information management platform or tool, configurable to each organization (company, facility or other entity for which screening audiograms are conducted), to automate the process of managing audiometric data, including scheduling, data collection and organization, statistical analysis, interpretation, reporting, followup tasks, and documentation.

There is a still further need for a system and method of analyzing audiometric data to provide an indication of the onset of NIHL in a subject or a population at a point prior to the disease being irreversible, in order to predict and reduce the risk for development of NIHL through noise exposure controls and other preventive measures.

There remains yet a further need for a system and method of analyzing audiometric data to distinguish NIHL form other types of hearing loss.

## SUMMARY OF THE INVENTION

The invention is as set out in the appended claims.

Other features and advantages of the present invention will be understood by reference to the detailed description and the examples that follow.

## BRIEF DESCRIPTION OF THE DRAWINGS

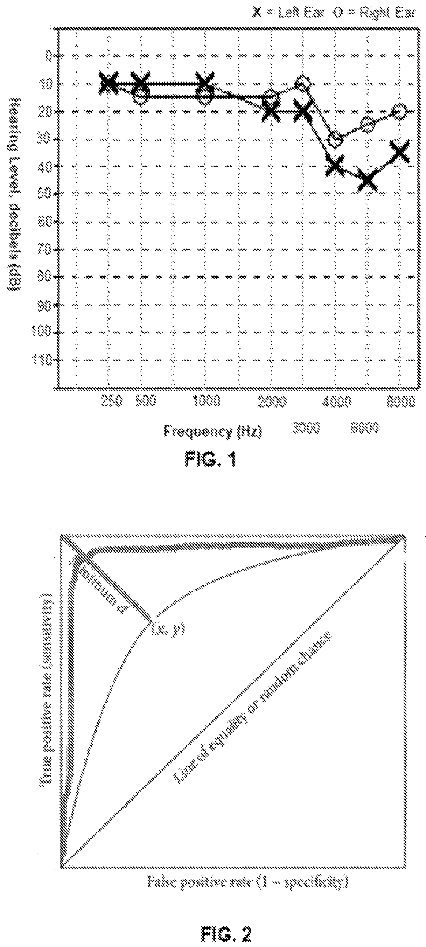

**FIG. 1** is a graph of an audiogram with a typical NIHL pattern. Data for both ears is indicated ("X" for the left ear, "O" for the right ear), with the minimum (0 dB) threshold located at the top and the physiologically maximum (120 dB) threshold at the bottom. The y-axis represents sound intensity in dB, and the x-axis represents frequency in Hz.

**FIG. 2** is a receiver operating characteristics ("ROC") curve. The x-axis is the false positive rate (1-specificity). The y-axis is the true positive rate (sensitivity). The bold line hugging the y-axis and then sharply bending to hug the top of the diagram represents a theoretically optimized, or "perfect" test, i.e., approaching 100% sensitivity and 100% specificity. The diagonal line running from the x-y axis intersection in the lower left to the upper right represents the "line of equality" or random chance. The curve in between these two represents what an actual ROC curve would look like. A diagonal line drawn from the upper left to this curve, represented by "d" in the diagram, represents specificity; the point where this line intersects the curve is where sensitivity and specificity are both maximized.

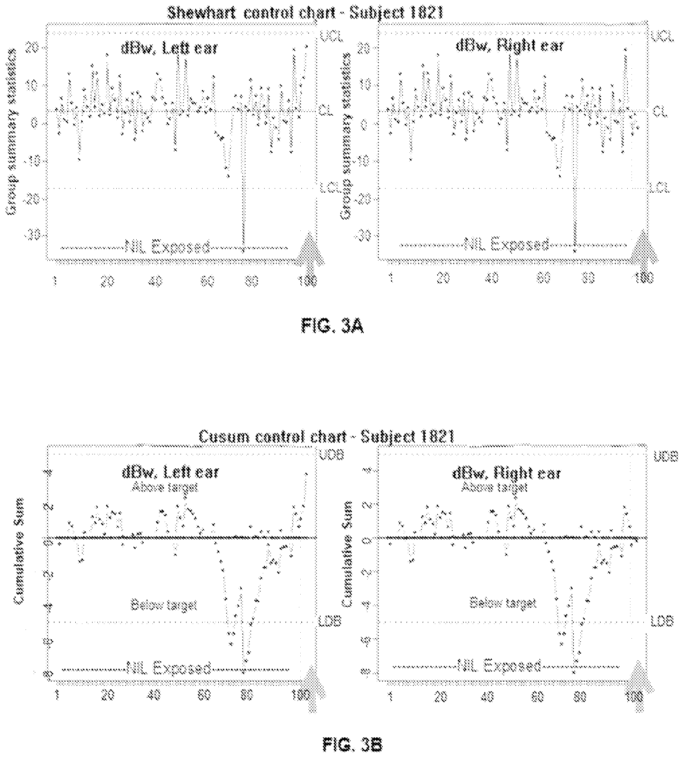

FIG. 3A is a Shewhart control chart for subject 1821 versus control employees. The x-axis represents the employees, whereas the y-axis represents $dB_w$ values for each employee-test. The vertical arrow at the right indicates the data for subject 1821 in the right ear (right panel) and left ear (left panel). Values to the left of the vertical line represent all data for employees in the 'control' SEG (NIL exposure, < 80 dB) group, from which the UCL (upper control limit) value was derived.

FIG. 3B is a cumulative sum (cusum) control chart for subject 1821 versus control employees. The x-axis represents the employees, whereas the y-axis represents cumulative positive or negative changes in $dB_w$ for those employees that fall above or below the target, respectively. The arrow indicates the data for subject 1821 in the right ear (right panel) and left ear (left panel). Values to the left of the vertical line represent all data for employees in the 'control' SEG (NIL exposure, < 80 dB) group, from which UDB (upper data boundary) value and LDB (lower data boundary) were derived.

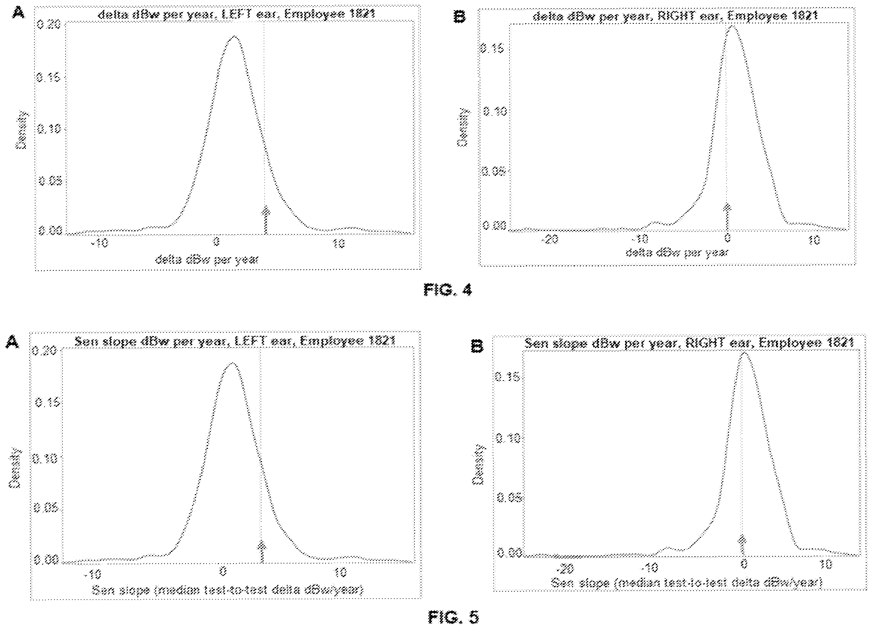

**FIG. 4** is a $\Delta dB_w$ comparison report graph for an individual employee. The arrow indicates where employee no. 1821's calculated WTS ($AdB_w$/year) stands in comparison to the rest of his SEG. The analysis for the left ear is shown in Panel A, and the analysis for the right ear is shown in Panel B. The x-axis represents the change in $dB_w$ per year, whereas the y-axis represents density of observations, represented by a smoothed histogram.

**FIG. 5 is** a Sen slope (annualized trend, computed as median test-to-test $AdB_w$/year) comparison report graph for an individual employee. The arrow indicates where employee no. 1821's annualized trend stands in comparison to the rest of his SEG. The analysis for the left ear is shown in Panel A, and the analysis for the right ear is shown in Panel B. The x-axis represents the Sen slope, whereas the y-axis represents density of observations, represented by a smoothed histogram.

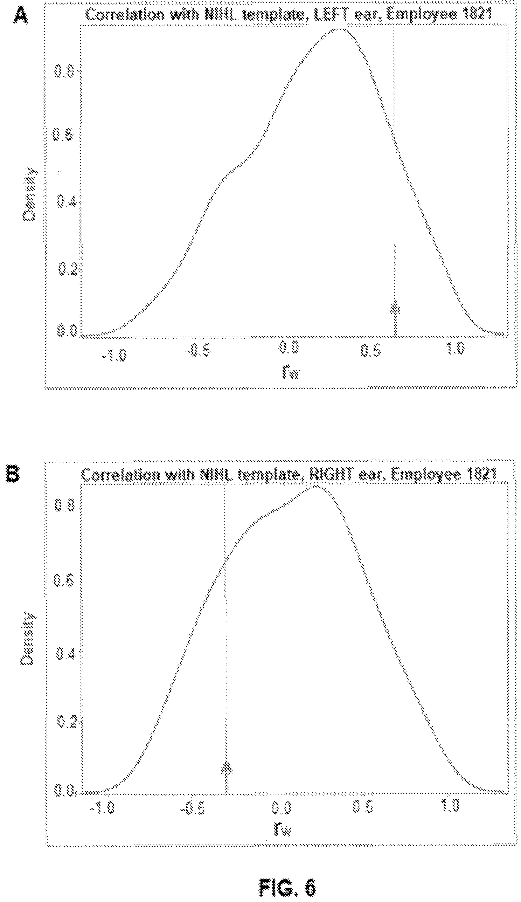

**FIG. 6** is a Weighted Correlation Coefficient ($r_w$, change in pattern toward early NIHL) comparison report graph for an individual employee. The arrow indicates where employee no. 1821's most recent $r_w$ value stands in comparison to the rest of his SEG. The analysis for the left ear is shown in Panel A, and the analysis for the right ear is shown in Panel B. The x-axis represents $r_w$, whereas the y-axis represents density of observations, represented by a smoothed histogram.

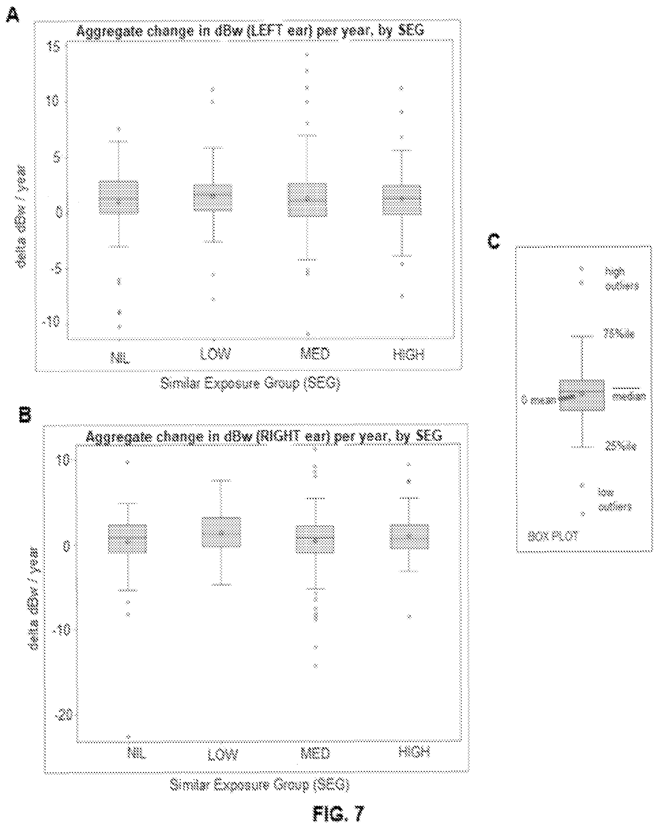

**FIG. 7** is side-by-side box plots of the aggregate $\Delta dB_w$/year for each SEG for both the left ear (Panel A) and right ear (Panel B). The legend for the box plot distribution is shown in Panel C. There are four (4) SEGs (NIL, LOW, MEDium, and HIGH) which together comprise a total N=456 employees with ≥2 tests per person.

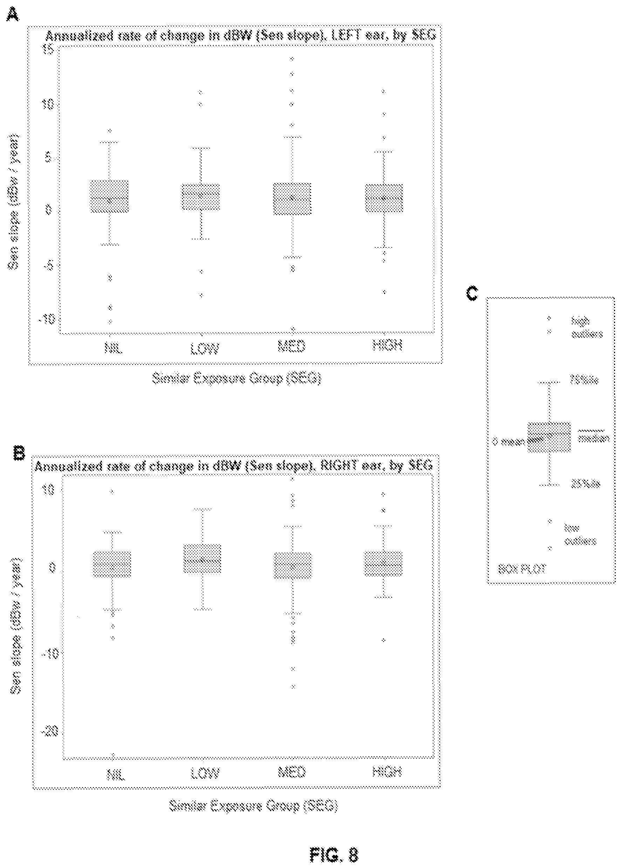

**FIG. 8** is side-by-side box plots of the aggregate Sen slopes (pooled annualized trends of median test-to-test $\Delta dB_w$/year) for both the left ear (Panel A) and right ear (Panel B). The legend for the box plot distribution is shown in Panel C. There are four (4) SEGs (NIL, LOW, MEDium, and HIGH) which together comprise a total N=456 employees with ≥2 tests per person.

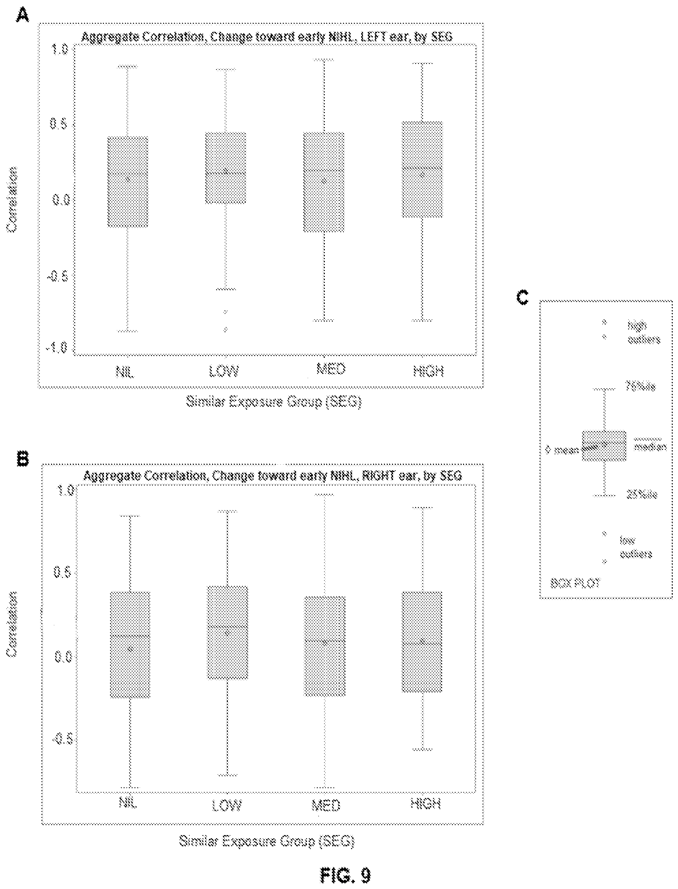

**FIG. 9** is side-by-side box plots of the aggregate Weighted Correlation Coefficients ($r_w$) values (pooled correlation of change toward early NIHL) by SEG for both the left ear (Panel A) and right ear (Panel B). The legend for the box plot distribution is shown in Panel C. There are four (4) SEGs (NIL, LOW, MEDium, and HIGH) which together comprise a total N=456 employees with ≥2 tests per person.

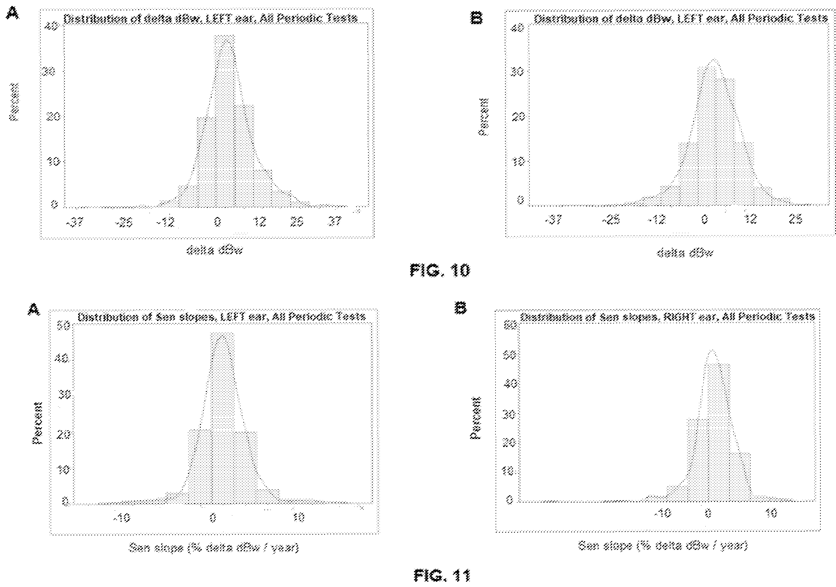

**FIG. 10** are graphs displaying the aggregate frequency distribution (density plot) of $\Delta dB_w$ (current vs. baseline test) for all employees with ≥2 tests per person (N=456 employees). The frequency distribution for the left ear is displayed in panel A, and the frequency distribution for the right ear is displayed in panel B. The x-axis represents $\Delta dB_w$, whereas the y-axis represents the proportion (percentage) of the population.

**FIG. 11** are graphs displaying the aggregate frequency distribution (density plot) of Sen slopes (pooled median test-to-test $\Delta dB_w$/year) for all employees with ≥2 tests per person (N=456 employees). The frequency distribution for the left ear is displayed in panel A, and the frequency distribution for the right ear is displayed in panel B. The x-axis represents Sen slope (median $\Delta dB_w$/year), whereas the y-axis represents the proportion (percentage) of the population.

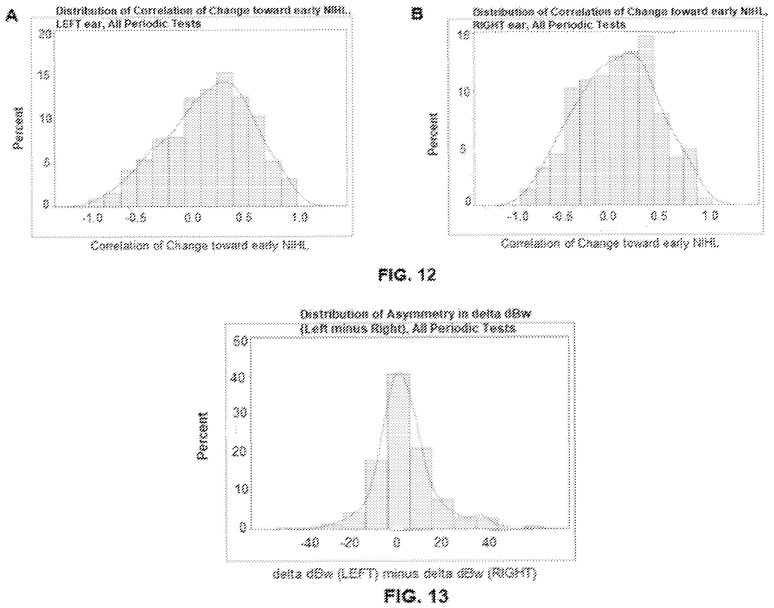

**FIG. 12** are graphs displaying the aggregate frequency distribution (density plot) of Weighted Correlation Coefficients ($r_w$) for all employees with ≥2 tests per person (N=456 employees). The frequency distribution for the left ear is displayed in panel A, and the frequency distribution for the right ear is displayed in panel B. The x-axis represents Weighted Correlation Coefficients, whereas the y-axis represents the proportion (percentage) of the population.

**FIG. 13** displays an aggregate frequency distribution (density plot) of the difference ($\Delta dB_{w\ Left}$ minus $\Delta dBw_{Right}$ for most recent test) as a measure of asymmetry for all employees with ≥2 tests per person (N=456 employees). The x-axis represents the difference between the left ear $\Delta dB_w$ and right ear $\Delta dB_w$, whereas the y-axis represents the proportion (percentage) of the population.

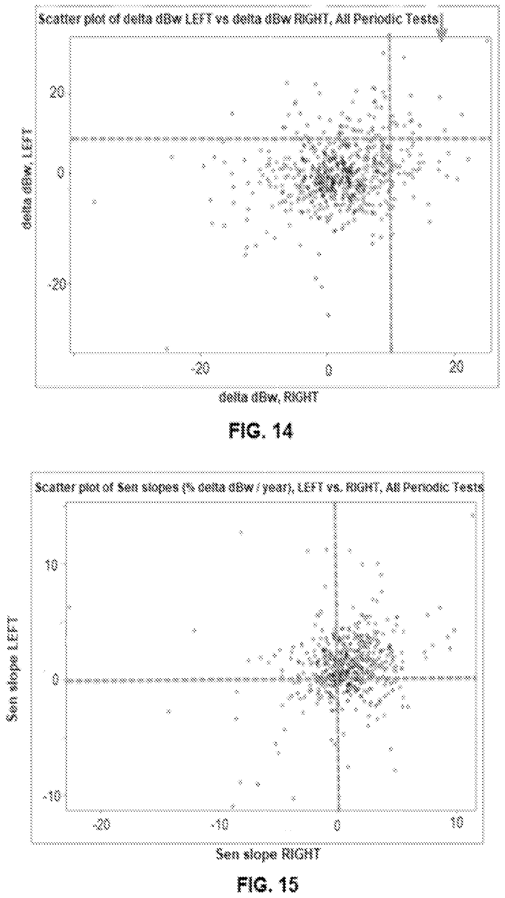

**FIG. 14** is an aggregate scatter plot of $\Delta dB_w$ in the left ear (y-axis) vs. right ear (x-axis) as a measure of asymmetry for all employees' with ≥2 tests per person most recent test. The dashed lines correspond to values (cutoffs) of 10 dB, the STS-OSHA definition (criterion).

**FIG. 15** is an aggregate scatter plot of Sen slopes (median $\Delta dB_w$/year, test-to-test) in the left ear (y-axis) vs. right ear (x-axis) for all employees' with ≥2 tests per person most recent test. The slopes can be positive or negative. The intersection of "0" Sen slope dashed lines indicates perfect symmetry with regard to annualized trend toward Early NIHL

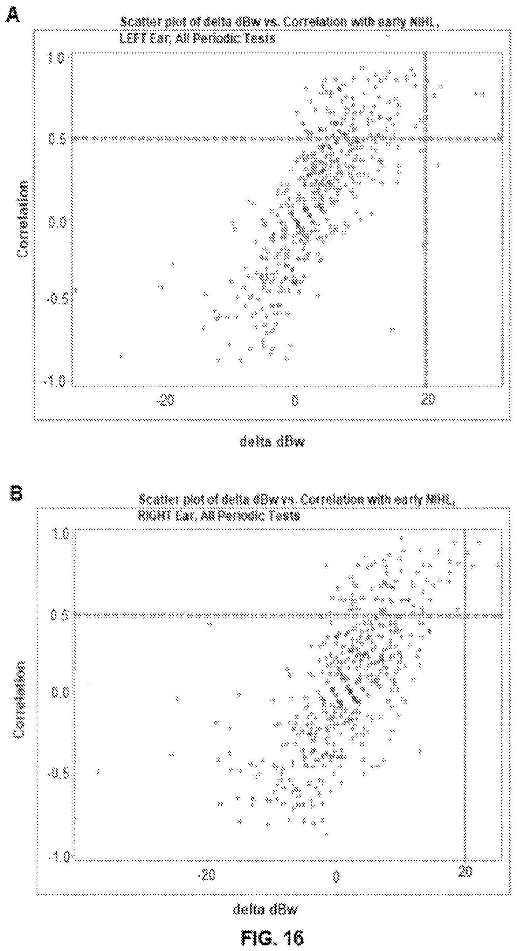

**FIG. 16** are scatter plots of $\Delta dB_w$ vs Weighted Correlation Coefficient ($r_w$) in both the left ears (Panel A) and right ears (Panel B) for all employees' with ≥2 tests per person most recent test. The horizontal line value is set to identify the early NIHL pattern ($r_w > 0.5$). The vertical line value is (arbitrarily) set to $\Delta dB_w = 20$ to maximize specificity. The values in the upper right-hand quadrant of Panel A and Panel B correspond to the employees with the highest probability of having Early NIHL.

## DETAILED DESCRIPTION OF ILLUSTRATIVE EMBODIMENTS

The present invention springs in part from the need for and development of a robust metric directed to transforming raw audiometric data for the purpose of obj ective, accurate, specific detection and prediction of particular types of progressive hearing deficiencies and diseases, such as early NIHL, that are diagnosed and characterized by a series of audiograms taken at intervals over a discrete time period, typically annually.

For purposes of this document and for clarity, all percentages referred to herein are percentages by weight (wt. %) or proportion of observations or number of persons, unless otherwise noted.

Ranges, if used, are used as shorthand to avoid having to list and describe each and every value within the range. Any value within the range can be selected, where appropriate, as the upper value, lower value, or the terminus of the range.

The term "about" refers to the variation in the numerical value of a measurement, e.g., temperature, weight, percentage, length, concentration, and the like, due to typical error rates of the device used to obtain that measure. In one embodiment, the term "about" means within 5% of the reported numerical value; preferably, the term "about" means within 3% of the reported numerical value.

As used herein, the singular form of a word includes the plural, and vice versa, unless the context clearly dictates otherwise. Thus, the references "a", "an", and "the" are generally inclusive of the plurals of the respective terms. Likewise, the terms "include", "including" and "or" should all be construed to be inclusive, unless such a construction is clearly prohibited from the context. Similarly, the term "examples," particularly when followed by a listing of terms, is merely exemplary and illustrative and should not be deemed to be exclusive or comprehensive.

The term "comprising" is intended to include embodiments encompassed by the terms "consisting essentially of' and "consisting of". Similarly, the term "consisting essentially of" is intended to include embodiments encompassed by the term "consisting of".

Various publications, including patents, published applications and scholarly articles, are cited throughout the specification.

As noted above, an audiogram is a test that measures the ability of a subject to hear a pure tone at standardized, ordinally ranked frequencies by air conduction. Suitable frequencies for use herein range from about 0.5 kilohertz (kHz) to about 10 kHz or more, e.g., 0.5 kHz, 0.6 kHz, 0.7 kHz, 0.8 kHz, 0.9 kHz, 1 kHz, 1.1 kHz, 1.2 kHz, 1.3 kHz, 1.4 kHz, 1.5 kHz, 1.6 kHz, 1.7 kHz, 1.8 kHz, 1.9 kHz, 2 kHz, 2.1 kHz, 2.2 kHz, 2.3 kHz, 2.4 kHz, 2.5 kHz, 2.6 kHz, 2.7 kHz, 2.8 kHz, 2.9 kHz, 3 kHz, 3.1 kHz, 3.2 kHz, 3.3 kHz, 3.4 kHz, 3.5 kHz, 3.6 kHz, 3.7 kHz, 3.8 kHz, 3.9 kHz, 4 kHz, 4.1 kHz, 4.2 kHz, 4.3 kHz 4.4 kHz, 4.5 kHz, 4.6 kHz, 4.7 kHz, 4.8 kHz, 4.9 kHz, 5 kHz, 5.1 kHz, 5.2 kHz, 5.3 kHz, 5.4 kHz, 5.5 kHz, 5.6 kHz, 5.7 kHz, 5.8 kHz, 5.9 kHz, 6 kHz, 6.1 kHz, 6.2 kHz, 6.3 kHz, 6.4 kHz, 6.5 kHz, 6.6 kHz, 6.7 kHz, 6.8 kHz, 6.9 kHz, 7 kHz, 7.1 kHz, 7.2 kHz, 7.3 kHz, 7.4 kHz, 7.5 kHz, 7.6 kHz, 7.7 kHz, 7.8 kHz, 7.9 kHz, 8 kHz, 8.1 kHz, 8.2 kHz, 8.3 kHz, 8.4 kHz, 8.5 kHz, 8.6 kHz, 8.7 kHz, 8.8 kHz, 8.9 kHz, 9 kHz, 9.1 kHz, 9.2 kHz, 9.3 kHz, 9.4 kHz, 9.5 kHz, 9.6 kHz, 9.7 kHz, 9.8 kHz, 9.9 kHz, 10 kHz, or more. Preferably, the range is between about 0.5 kHz and about 8 kHz. Suitable ordinally-ranked frequencies are, e.g., 0.5 kHz, 1, kHz, 2, kHz, 3 kHz, 4 kHz, 5 kHz, 6 kHz, 7 k Hz, and 8 kHz, it being understood that other ordinally-ranked frequency values can be selected for conducting audiograms. The hearing ability of the subject for each frequency, at various volumes (amplitudes), may be measured in each ear and recorded in decibels (dB). An audiogram may be generated by using an audiometer, which is a device regularly used by audiologists, physicians, and other hearing professionals for evaluating hearing acuity, according to specified methods for calibration and accuracy.

Audiometers can be a self-contained hardware unit comprising a sound generating unit connected to a pair of headphones, or software-based programs run through a computer connected to headphones. Preferably, audiograms are conducted in a sufficiently quiet environment, typically a soundproof booth designed for this purpose. Examples of audiometers include, without limitation, audiometers produced by Welch Allen, Benson Medical, Primus, Maico, Grason-Stadler, and others.

When audiograms are performed, the hearing levels, or thresholds, at each sound frequency are recorded separately for each ear in decibels in interval (i.e., non-continuous) increments of 5 dB. Individual audiogram raw data is typically reported in tabular format, and it is commonly graphically displayed as an inverted line with markers of the hearing thresholds on the y-axis for each of the measured frequencies along the x-axis. The hearing levels (results) can either be reported in a separate graph for each ear, or both ears can be plotted together on a single graph. The graphical reports typically use the convention of an "X" denoting the left ear and "O" denoting the right ear, with the minimum (typically 0 dB) threshold located at the top and the physiologically maximum threshold value (110 or 120 dB) at the bottom.

The raw audiometric data may then be transformed into robust metrics that can be further subjected to post hoc statistical analysis. For instance, the audiogram measurement data can be used to calculate the Weighted Hearing Level (W) metric, which summarizes the magnitude of hearing loss in an ear specifically toward NIHL, and is derived by mathematically transforming raw audiometric data from one ear into a single number (vector) that is expressed in $dB_W$ units which are equivalent to the units (dB) in which the raw data are obtained. In this document, the W is sometimes referred to herein as "W ($dB_W$)."

In addition to W, provided herein are several derived metrics including the Weighted Threshold Shift (WTS), the Weighted Correlation Coefficient ($r_w$), and the Weighted Left Right Laterality ($W_{L-R}$). The WTS reflects the magnitude and direction of change in hearing in an ear (in $dB_W$ units) specifically toward (or away from) NIHL between an audiogram obtained at a given time (the "current" test) relative to the baseline audiogram. In this document, the WTS is sometimes referred to as "WTS ($\Delta dB_W$)." The $r_w$ measures the extent of a linear relationship between a change in the WTS ($\Delta dB_W$) and the early NIHL template pattern. A similar correlation coefficient ($r_B$) can also be calculated for a baseline test's relationship to the early NIHL pattern. Finally, the $W_{L-R}$ measures the extent of asymmetry between Left and Right ears' ("sides") Weighted Hearing Level in an individual audiogram.

The W and its derived metrics (WTS, $r_w$, $W_{L-R}$) can be subjected to *post hoc* statistical tests that would not otherwise be feasible using raw audiometric data, and which thereby allow audiometric data to be utilized to:

1. Accurately predict (early) NIHL before a STS occurs, with higher sensitivity and specificity for NIHL than the STS;

2. Distinguish (identify) individual and aggregate early NIHL audiometric patterns (statistical "signal") from other hearing loss conditions (at baseline or subsequently arising), background or normal hearing patterns (statistical "noise") in the normal-to-mild hearing loss range before STSs occur. This capability is available for any type of normal or abnormal baseline audiogram test;

3. Evaluate the overall preventive effectiveness of a HCP, or comparative effectiveness among SEGs within a company or facility by comparing the measured trends, correlations and laterality, or combinations thereof, to numerical thresholds or rankings determined by theoretical, empirically observed, or consensus-based criteria;

4. Enable the trends in progression toward NIHL in and among individuals and groups over time to be quantified and compared to determine the effectiveness of hearing protection and noise exposure controls in preventing irreversible hearing loss (reflected by the STS); and

5. Accurately identify outlier results and their probable causation related to noise exposure in audiometrically screened populations.

To determine the Weighted Hearing Level (W) for each ear ($W_{Right}$ and $W_{Left}$) from raw audiogram data, the following calculations may be performed:

1. Establish the $f_i$ **values** to be used in the calculation, as described below (Table 3).

2. Calculate $\bar{f}$ = the average (mean) of the $f_i$ **values**. For $i = 7$, $\bar{f} = f_1 + f_2 + ... + f_7)/7$.

3. For each (i) test frequency, calculate $f_i - \bar{f}$ : = $f_1 - \bar{f}$, $f_2 - \bar{f}$,... $f_7 - \bar{f}$

4. Multiply $L_i$ by ($f_i - \bar{f}$) for each $i$ measured response and corresponding theoretical response: = $L_1 (f - \bar{f}) + L_2(f_2 - \bar{f}) + ... + L_7 (f_7 - \bar{f})$

5. Add (sum) all the $L_i$ x ($f_i - \bar{f}$) values from Step 4. This is the first numerator $\Sigma_i L_i(f_i - \bar{f})$.

6. For each *(i)* test frequency, calculate the square of ($f_i - \bar{f}$) : = ($f_1 - \bar{f}$)$^2$, ($f_2 - \bar{f}$)$^2$, ... ($f_7 - \bar{f}$)$^2$

7. Calculate $f_m$ by finding the largest (maximum) value among the $f_i$ values.

8. Subtract $\bar{f}$ from $f_m$: $f_m - \bar{f}$. This is the last factor (i.e., the second numerator).

9. Add (sum) all the ($f_i - \bar{f}$)$^2$ values from Step 6: = ($f_1 - \bar{f}$)$^2$ + ($f_2 - \bar{f}$)$^2$ + ... + ($f_7 - \bar{f}$)$^2$. This is the denominator $\Sigma_i (f_i - \bar{f})^2$.

10. Multiply the first and second numerators (Step 5 and 8), then divide this product by the denominator (Step 9).

11. Calculate $\bar{L} = \Sigma_i L/i$ where $i = 7$ (count of 500, 1000, 2000, 3000, 4000, 6000, 8000 Hz)

12. Add quotient from Step 10 to $\bar{L}$. Note: The quotient value may be positive, negative or zero.

A sample calculation of W for raw audiogram data (using the 4kHzV1 template, *see* Table 3) is provided in Table 1 below.

**TABLE 1. Calculation of Weighted Hearing Level (W) for a sample audiogram.**

| i | $L_i$ | $f_i$ | $f_i$ - $f_{avg}$ | $L_i$ x ($f_i$ - $f_{avg}$) | ($f_i$-$f_{avg}$)$^2$ | $dB_W$ |
|---|---|---|---|---|---|---|
| 0.5K | 5 | 0 | -0.79 | -3.93 | 0.62 | **24.6** |
| 1K | 10 | 0 | -0.79 | -7.86 | 0.62 | |
| 2K | 15 | 0.5 | -0.29 | -4.29 | 0.08 | |
| 3K | 10 | 1 | 0.21 | 2.14 | 0.05 | |
| 4K | 25 | 3 | 2.21 | 55.36 | 4.90 | |
| 6K | 15 | 1 | 0.21 | 3.21 | 0.05 | |
| 8K | 10 | 0 | -0.79 | -7.86 | 0.62 | |
| Avg | 12.86 | 0.79 | | | | |
| Sum | | | | 36.79 | 6.93 | |

(continued)

| i | $L_i$ | $f_i$ | $f_i - f_{avg}$ | $L_i \times (f_i - f_{avg})$ | $(f_i-f_{avg})^2$ | $dB_w$ |
|---|---|---|---|---|---|---|
| Max | | 3 | | | | |

To calculate the WTS ($\Delta dB_w$) for the current test in each ear:

1. Subtract $W_{Baseline\ Test}$ from $W_{Current}$ Test.

Table 2 demonstrates calculations of W and WTS using the 4kHzV1 template for a hypothetical audiogram (one ear) demonstrating a typical progression from normal (baseline) hearing to NIHL over 7 years. Table 3 enumerates the $f_i$ values for the 4kHzV1 template. The raw audiometric data for the current test could also be plotted in graphical format, as illustrated in Figure 1. As shown in Figure 1, there are 8 hearing frequencies recorded for each ear, with the lowest (250 Hz) not being clinically important and thus disregarded (i.e., $i$ = 7). The "notch" is V-shaped in each ear, and occurs in the Right ear at 4 kHz (4000 Hz) and in the Left ear at 6 kHz (6000 Hz). Both ears have a "recovery" at 8 kHz (8000 Hz).

**TABLE 2. Example Audiometric progression of NIHL (in one ear) and calculations of WTS and STS-OSHA.**

| | B | Year 1 | Year 2 | Year 3 | Year 4 | Year 5 | Year 6 | Year 7 |
|---|---|---|---|---|---|---|---|---|
| 0.5 kHz | 0 | 0 | 0 | 5 | 5 | 5 | 5 | 5 |
| 1 kHz | 5 | 5 | 5 | 10 | 10 | 15 | 10 | 10 |
| 2 kHz | 0 | 5 | 5 | 10 | 5 | 10 | 10 | 10 |
| 3 kHz | 5 | 5 | 10 | 10 | 10 | 10 | 5 | 10 |
| 4 kHz | 5 | 5 | 10 | 15 | 15 | 20 | 25 | 25 |
| 6 kHz | 5 | 10 | 5 | 10 | 10 | 15 | 15 | 15 |
| 8 kHz | 5 | 5 | 5 | 5 | 5 | 10 | 10 | 10 |
| 234 kHz Avg | 3.3 | 5.0 | 8.3 | 11.7 | 10.0 | 13.3 | 13.3 | 15.0 |
| STS (dB) | | 1.7 | 5.0 | 8.3 | 6.7 | 8.8 | **10.0** | 11.7 |
| W ($dB_w$) | 5.3 | 6.6 | 10.9 | 15.3 | 15.1 | 19.6 | 23.3 | 24.6 |
| WTS ($dB_w$) | | 1.3 | 5.6 | **10.0** | 9.8 | 14.3 | 18.0 | 19.3 |
| All calculations are rounded to one decimal place. | | | | | | | | |

The calculated values of WTS (in $dB_w$ units) using the 4kHzV1 template are compared to the corresponding calculated values of STS (in dB) for the same set of audiograms. The results are further explained below.

**TABLE 3 4kHzV1 ENIHL Template**

| $i$ | Freq (kHz) | $f_i$(4kHzV1) | $f_i$(4kHzV2) | $f_i$(4kHzV3) |
|---|---|---|---|---|
| 1 | 0.5 | 0.0 | 0.0 | 0.0 |
| 2 | 1 | 0.0 | 0.0 | 0.0 |
| 3 | 2 | 0.0 | 0.0 | 0.0 |
| 4 | 3 | 1.0 | 0.5 | 1.0 |
| 5 | 4 | 2.0 | 2.0 | 2.0 |
| 6 | 6 | 1.0 | 1.0 | 0.5 |
| 7 | 8 | 0.5 | 0.5 | 0.5 |

The $r_w$ and $W_{L-R}$ are calculated by following the mathematical operations as defined by the equations described above. In connection with the above, calculations do not require rounding, or they can be rounded to 1 or 2 decimals. In one embodiment, W (in $dB_w$ units) can be reported to one decimal place. It is also noted that W can have a positive, negative or zero value. Finally, W is reported in equivalent units of decibels (dB) which reflect the mathematical weighting of the hearing levels at each frequency, and therefore are termed "$dB_w$."

It will be understood that, to calculate the WTS ($\Delta dB_w$) for a periodic (current) test after an initial (baseline) test, one would subtract the (initial) "baseline" test W from the current test W for each ear, *i.e.,* $WTS_{Right} = W_{Right}$ Current - $W_{Right}$ Baseline and $WTS_{Left} = W_{Left}$ Current - $W_{Left\ Baseline}$, where "Left" means the left ear, and "Right" means the right ear. The WTS ($\Delta dB_w$) value can be positive, negative, or zero. A positive WTS means the hearing loss has increased (worsened) toward NIHL. A negative WTS means the hearing loss has decreased (improved) or changed its shape away from NIHL. A zero (0) WTS means no change in either direction.

It will be understood that the calculation of $r_w$ for a periodic (current) test after an initial (baseline) test produces a value between -1.0 and +1.0, and can be reported to one, two or more decimal places. A $r_w$ value close to -1.0 means a negative correlation of the audiometric results with early NIHL. A $r_w$ value close to +1.0 means a positive correlation of the audiometric results with early NIHL. For example, if a current (non-baseline) audiogram has a high WTS ($\Delta dB_w$), but it has a low correlation with the NIHL template, it indicates a substantial hearing loss, but one that is *not* consistent with NIHL.

It will be understood that the calculation of $W_{L-R}$ for a subsequent (current) test after an initial (baseline) test is based upon the absolute value of a difference between ears, and thereby produces a value between zero (0) and +1.0, which can be reported to one, two or more decimal places. A $W_{L-R}$ value close to or equal to 0 means no difference between sides, and a $W_{L-R}$ value close to or equal to 1.0 means completely different between sides. The convention to subtract the Right value from the Left value, rather than vice versa, is arbitrary; and because either the numerator and/or the denominator could be negative values, absolute values (|...|) are used to express the relative difference in $dB_w$ between the two ears.

For screening audiograms, i normally is equal to seven (the count of frequencies of 0.5, 1, 2, 3, 4, 6, 8 kHz), or eight (when 0.25 kHz is also included). However, i can be equal to any number of frequencies that are measured in an audiogram. For example, if lower, intermediate and/or higher frequencies are also measured, i would be greater than seven (or eight), and if only six frequencies are measured, *i* would be six. The preferred range of *i* is between six and ten. A more preferred range is between seven and eight.

The Weighted Hearing Level (W) is designed to closely summarize the hearing loss based on the relative magnitude of hearing levels at each frequency that correspond to a typical pattern, or "template" of NIHL at its earliest, recognizable and distinguishable form *(see* Table 3 above). This template is represented mathematically by a series of expected or modeled relative hearing levels denoted by the symbol $f_i$ for each $i$ frequency in the audiogram. The values of $f_i$ are unitless scalars that can be expressed as integers or to one or more decimal places. For purposes of simplicity, they are described herein and employed in the examples rounded to increments of 0.5. For early NIHL, the relative values of $f_i$ are established to resemble the expected, or hypothetical, pattern of NIHL's characteristic high frequency "notch" at the earliest audiometrically recognizable stage of disease, preferably at or before an STS has occurred (Table 1). However, the particular absolute or relative values or combinations of $f_i$ are not fixed, nor are they necessarily based on any particular empirical or consensus-based audiometric diagnostic criteria for the reasons discussed above; namely, 1) the shape of the NIHL audiogram is inherently variable among any two subjects even with its characteristic high frequency "notch" (which is typically "V" shaped with a single nadir frequency but can also be "U" shaped with two equal nadir frequencies); and 2) there is no single, absolute or universally accepted clinical definition of what audiometric pattern constitutes NIHL at its earliest detectable phase, or what that pattern is when a STS is detected.

The flexibility of the expected response functions $f_i$ allows for the W, WTS and other metrics to be used to evaluate common variants of NIHL, such as the 4 kHz peak (most common) or the 6 kHz peak, or the less common but recognized 3 kHz peak. The weighted expected response functions $f_i$ can be modified to fit the patterns typically observed at a given company, occupation or type of noise exposure. Several variations of expected response ($f_i$) template patterns (indicating early NIHL) can be utilized, e.g., 4kHzV, 6kHzV, 4-6kHzU, modelled after the most commonly recognized patterns of NIHL in its early stages with peak hearing loss at 4 kHz (V-shaped), 6 kHz (V-shaped), or both 4 and 6 kHz (U-shaped), respectively. For each such variation, sub-variations ("sub-variants") reflecting asymmetry can also be used. Examples of three sub-variants of the 4kHzV template (labelled as sub-variants 1, 2 and 3) are shown in Table 3 above. All three sub-variants have the same V-shaped pattern with subtle differences in notch symmetry: $f_i$ (4KHzV1) is symmetrically weighted around the 4 kHz notch, whereas $f_i$ (4kHzV2) and $f_i$ (4KHz3V) are asymmetrically weighted around the 4 kHz notch.

In addition to the aforementioned purpose and functionality, attributes of the Weighted Hearing Level (dBw) as a metric for interpretation of audiometric data include the following advantages.

- The W (dBw) method produces a value that is expressed in the same data measurement units (dB) as the raw audiometric data. The W (dBw) metric can therefore be statistically analyzed and reported in units already familiar to those skilled in the art of audiometric testing, interpretation, and compliance. Age-adjustment factors, such as those found in the OSHA and MSHA Standards, can be applied.
- The weighted expected response functions $f_i$ can be modified to fit the patterns typically observed at a given company, occupation or type of noise exposure. The sensitivity and specificity for detection can be determined by comparing results to actual audiograms of workers with or without an STS or clinically defined NIHL over a period of time, and can be compared to one another using ROC curves. An exemplary ROC curve is shown in Figure 2. The area under the curve (AUC) represents the relative degree of separation between two outcomes: a perfectly accurate test has AUC = 1.0 and a random test has AUC = 0.50. An AUC of 0.7, for example, represents a 70% probability that model will be able to distinguish between positive class and negative class.

- Correlation coefficients (separate from $r_w$) can be applied to evaluate how closely the $dB_W$ differentiates NIHL from the characteristic audiometric patterns of other medical conditions that cause hearing loss.
- The W metric ($dB_W$) can be normalized to fit any set of data through the design and use of the weighted expected response functions $f_i$. Normalization means adjusting values measured on different scales to a notionally common scale. That is, for the same relative magnitude of values selected for $f_i$, the W metric will not be affected.
- Each ear (left and right) can be analyzed separately, or the two values can be averaged to combine them into a single metric. This capability recognizes that for noise exposure, there are two ears exposed, but only one subject (person) whose overall hearing is affected.

The WTS ($\Delta dB_W$) is defined as the arithmetic difference ("Δ") between the $dB_W$ value of the current audiogram minus the $dB_W$ value of the baseline audiogram. The WTS is a readily calculated metric that measures the magnitude and direction of overall changes in hearing loss towards early NIHL in a subject from baseline test to current test, analogous to how the (non-specific, non-predictive) regulatory STS is defined. For an individual subject, the calculated WTS ($\Delta dB_W$) for each subsequent audiogram can be computed to evaluate, tabulate and plot changes over time. Like for the STS, an absolute WTS ($\Delta dB_W$) cutoff or criterion value could be established as a predictive tool, as well as to identify potential outliers or individuals who require investigation or intervention, such as exposure controls or work practices, or even temporary or permanent removal from excessive noise exposure. An appropriate, comparable criterion or "cutoff" for the WTS could be $\geq 10$ $dB_W$, which corresponds to the STS-OSHA- or STS-CSA-defined criterion of $\geq 10$ dB.

In addition to the aforementioned purpose and functionality, attributes of the WTS ($\Delta dB_W$) as a metric for statistical analysis of audiometric data include the following:

- The WTS ($\Delta dB_W$) metric adjusts for abnormal baseline tests (and any underlying cause of hearing loss) since it measures the change specifically toward a NIHL pattern.
- The analysis of trend in WTS ($\Delta dB_W$) for individuals or groups does not require a control population or data set. Control data for occupational audiometry is not routinely collected in industry, and reliable control data sets are not publicly available.
- The WTS method is not adversely affected by the effects of any pre-existing hearing loss (e.g., occupational or non-occupational NIHL, presbycusis, tympanosclerosis) that may have preceded the employee's baseline test, date of hire, or date of entry into the HCP.
- Analysis can be performed on WTS ($\Delta dB_W$) for either ear or combined ears for any individual person. Differences in trends toward unilateral (one ear) versus bilateral (both ears) hearing loss can also be performed using the same methods.
- By utilizing the baseline test as the subtrahend (the number that is subtracted) rather than the previous test (for cases where there are more than two tests), the WTS ($\Delta dB_W$) minimizes the signal-to-noise ratio inherent with test-to-test variability.
- The WTS ($\Delta dB_W$) can be analyzed in relatively small groups of subjects, and can be reliably computed on a variable number of serial audiograms. These conditions apply to the many noise-exposed workers who are employed in small-to-medium-sized companies.
- Beyond its utility for purposes of evaluating individual audiogram changes toward NIHL, WTS can be applied to aggregate data analysis, as described below.

Other derived summary statistics from the W (dBw) and WTS ($\Delta dB_W$), such as change in W ($dB_W$) between the two most recent (periodic) tests or between *any* two tests, percent change in W ($dB_W$) per unit time, or variation in W ($dB_W$) as measured by the absolute value of a pairwise difference, can be similarly calculated and can then be analyzed and compared using a variety of either parametric or non-parametric statistical methods, as described below.

For all such analyses, the time period could be measured in calendar time (in years or months) or the relative duration between periodic tests (e.g., third year to fifth year) in the HCP, or duration of noise exposure or employment. All such analyses can evaluate effects in a given ear (Left or Right), or both ears by presenting the data from each ear and comparing them side-by-side, or by combining them into a single metric, such as an arithmetic average, as described above.

*Statistical Analysis of Individual Trends*

Short-term (year-to-year or test-to-test) changes in W ($dB_W$) and WTS ($\Delta dB_W$) in an individual subject can be graphically plotted using standard statistical tools such as a control chart, also known as a Shewhart chart *(see, e.g.,* Figure 3). Shewhart charts are used to plot acute changes and identify outliers in approximately normally distributed data. The upper and lower confidence limits (UCL and LCL) are 3 standard deviations around a population mean (average). In the analysis of W ($dB_W$) metrics, values that are unexpectedly high relative to the reference (baseline) test are of interest. The LCL does not have relevance for audiometry because hearing levels tend to increase (worsen) or stay the same, but do not improve over time. Thus, the analyses use one-sided control limits, flagging elevated values that are above the upper confidence limit.

For more slowly developing individual audiometric changes over a number of years, statistical tools such as a cumulative sum control chart, also known as a cusum chart, can be utilized to plot temporal distributions *(see, e.g.,* Figure 1 in the Example). A cusum chart monitors gradual changes ("drift") in observed levels by calculating the cumulative sum of deviations from a target-which in this case is zero (0) change in $dB_W$. A cusum chart is defined by accumulating a positive sum based on observations that are appreciably above an upper data boundary (UDB), while a cumulative negative sum is calculated based on observations that are appreciably below the reference level (lower data boundary, LDB), which represent 5 standard deviations above or below 0, respectively. The cusum chart identifies an out-of-control process by an upward or downward drift of the cumulative sum until it crosses the boundary. An assignable cause is suspected whenever the cusum chart indicates an out-of-control process.

Either the Shewhart chart or the cusum chart can utilize the entire employee population, the subject's SEG, or any other defined sub-population to compute the upper and lower control limits (UCL and LCL) or data bounds (UDB and LDB), respectively. In addition, individual trends for a particular worker can be directly compared to one or more applicable aggregate (group) trends through the statistical methods described below.

Table 2 above illustrates a classic hypothetical audiogram progression from normal hearing to the characteristic high frequency notch with a peak at 4 kHz. This hypothetical case demonstrates audiogram progression in one ear from "normal" at baseline (B) to NIHL over 7 years. The STS ($\Delta 234$ kHz average $_{Current-Baseline} \geq 10$ dB) is detected at Year 6, whereas the WTS ($W_{Current} - W_{Baseline} \geq 10$ $dB_W$) for the same audiogram is detected at the Year 3 test.

The real-world study (Examples 1 and 2) of the invention described below provides additional examples of how the application of the weighted model to generate a WTS reveals early NIHL significantly before NIHL is detected by a STS-OSHA or CSA-STS.

*Statistical Analysis of Aggregate Trends and Distributions*

The W and its related, derived metrics (WTS, $r_W$, $W_{L-R}$) are most powerful in the analysis of aggregate audiometric data involving groups of workers (e.g., SEGs) to predict and measure trends that cannot otherwise be accomplished through interpretation of raw individual audiometric data or use of STS values.

Both parametric and non-parametric statistical tests can be applied to W and its related, derived metrics (WTS, $r_W$, $W_{L-R}$). Non-parametric methods have important advantages in the application to workplace populations because they obviate the need to assume a normal distribution of data, can be utilized with relatively small groups, are less impacted by missing values and uneven time intervals, and are not as susceptible to the effects of outliers.

A robust, non-parametric test for measuring gradual rates of progression of the response (hearing loss) over time is the Sen-Theil slope, also known as a Theil-Sen estimator or Sen slope estimator. This "runs test" estimates a (linear) slope as the median of all of the pairwise slopes between data points with respect to time. It is a useful summary statistic for identifying and quantifying an increasing or decreasing trend in such a way that occasional outliers do not adversely affect the calculation. The Sen slope is defined as the median of the pairwise slopes between points (e.g., test-to-test) for a subject over a defined time interval, where the denominator is typically a unit of time (e.g., years). The time interval between pairs of data points does not need to be constant-an advantage for samples that are not always collected at precise time intervals, or where there are missing values. The Sen slope can be tested for significance (i.e., whether the slope is different from zero or some other specified threshold), producing a z score and a corresponding p value (probability due to chance) which is reported with 95% upper and lower confidence intervals for the median slope. Thus, if trends in individual or aggregate $dB_W$ (and $\Delta dB_W$) values are increasing over a defined time period, the slope will be positive; if trends are decreasing over time, the slope will be negative. When the $dB_W$ data are natural log-transformed, Sen's slope can be interpreted as a proportional rate of change in NIHL per unit time (e.g., $\% \Delta dB_W$ per year), which explains not only whether a change is significant, but also how large it is.

The Sen slopes of all subjects tested in a group (e.g., each SEG) in a given time period can be pooled (compiled), and their distributions and measures of central tendency are measured by calculating the mean or median value for each group (SEG). The mean or median for each group can then be compared to one another for a given time period, or compared to themselves before versus after a noise exposure control intervention (e.g., reduce noise sources or upgrade HPDs) has been implemented, using *post-hoc* non-parametric tests and plotted graphically as follows. A standard statistical test such as analysis of variance (non-parametric one-way ANOVA by ranks tests to compare medians) yields a standard probability (*p*) value and confidence intervals (typically 95th percentile or 99th percentile), which can be interpreted to make decisions about the magnitude and direction of the findings.

- The Kruskal-Wallis (KW) test is a non-parametric one-way analysis of variance (ANOVA) by ranks which compares multiple (3 or more) sample population medians, such as SEGs. Like other non-parametric tests, it does not require the populations from which the samples are drawn to have normal distributions or equal variancesassumptions that parametric tests typically require. The KW test requires at least ordinal data, and uses information to determine whether or not the observations are above or below a single number. The KW test (2-tailed) is used to compare whether median values ($dB_W$, $\Delta dB_W$, Sen slope, $r_W$) are the same (null hypothesis) or different among 3 or more SEGs. A *p* value (but not confidence intervals) is produced.
- The Mann-Whitney (MW) test is used to determine if there is a significant difference of the medians between two groups (e.g., SEGs), with a null hypothesis that the median of one group is larger than the other. The MW is applied herein to compare each pair of groups when the KW test is positive. The pairwise MW test (one-tailed) test produces a test statistic T, a *p* value and confidence intervals. When multiple comparisons are required from the finding of a significant difference in the KW between a set of 3 or more medians, a Tukey-Cramer adjustment can be applied to reduce the accumulation of false positives due to multiple comparisons.

The comparative findings can be graphically displayed as a box plot (also known as a box and whiskers plot, *e.g.,* as shown in Figure 7) with the computed statistic and *p* value of the Kruskall Walls or Mann Whitney tests to accompany it.

For an individual subject, the Sen slope can be compared to values within the same individual or SEG, the entire population, or any defined sub-population (a "you are here" snapshot analysis) within a given time period or employment or exposure duration by plotting values as a histogram or smoothed density curve *(see, e.g.,* Figure 4).

Alternative statistical methods are available for these analyses. For example, an overall slope within an individual or group can also be calculated as a pooled Kendall's tau-beta. A parametric ANOVA could be utilized instead of its non-parametric counterpart. Other non-parametric methods for temporal trends of aggregate audiometric summary statistics (WTS) or for a given individual person's audiograms include the Wald-Wolfowitz or the Wallis-Moore runs test.

All of these statistical analyses can be applied to audiometric data for one ear (such as all left ears or all right ears) or both ears. Since hearing loss typically remains unchanged or progresses and only rarely improves in adults, and since the primary outcome of interest is whether and how much increase in hearing loss has occurred as reflected by W (dBw) or WTS ($\Delta dB_W$), the statistical test should be preferably be one-tailed, with the exception of the KW test and any other tests explicitly recommended to be two-tailed.

*Subgroup comparisons*

WTS ($\Delta dB_W$) trends over time, both among and across the members (workers) of each SEG and as well as for an individual person or ear (see above) within a SEG, can be analyzed with statistical tests to quantify the magnitude and direction by which audiometric results change over time.

The distribution of WTS ($\Delta dB_W$) or Sen slopes (median $\Delta dB_W/\Delta time$) for a selected time period, duration of employment or noise exposure (e.g., duration of time in the HCP) can also be compared across SEGs using histograms or density plots for either or both ears *(see, e.g.,* Figure 10). These analyses estimate the extent to which aggregate audiograms are changing toward (early) NIHL over time.

*Asymmetry Analysis*

Most NIHL is a bilateral (both ear) process, whereas other forms of hearing loss can be asymmetric or involve only one ear. However, progression of NIHL is rarely perfectly symmetrical as measured by audiograms. By utilizing the WTS to compare $\Delta dB_W$ in Right versus Left ears (sides) and comparing them in a scatter plot, criteria can be set to identify outliers *(see, e.g.,* Figure 14). The distribution of symmetrical vs. asymmetrical changes can be plotted as a histogram or density plot by placing zero (0) difference point in the center of the x-axis. In occupational populations where asymmetric NIHL is expected (e.g., police officers and military who regularly use firearms on one side of the body), the $W_{L-R}$ metric can be utilized to assess the extent of asymmetric hearing loss, and estimate the proportion of such incidences plausibly attributable to workplace noise exposure.

*Outlier Analysis*

By comparing the WTS ($\Delta dB_W$) to the $r_W$ for a group such as the entire worker population or a particular SEG, and plotting the data as a scatter plot and then setting a criterion (i.e., a cutoff, depending on desired sensitivity and specificity), the proportion and subject identities of outlier audiograms indicative of significant changes toward early NIHL can be statistically identified *(see, e.g.,* Figure 16). As discussed above, these analyses can be made for either ear (Left or Right), both ears, or a summary metric such as the average W (dBw) or WTS ($\Delta dB_w$) for Left and Right ears.

*Data Collection, Analysis, Interpretation, and Reporting*

The aforementioned data collection, organization, calculations and statistical analyses are typically conducted using computerized systems and software designed for this purpose. In theory, these data could be managed manually on paper or manually recorded using desktop or web-based software applications such as spreadsheets, including Microsoft Excel, Google Sheets, Apache Open Office, Quattro Pro, and the like. The calculations could similarly be performed by hand or by using spreadsheets with customized formulas, and could be programmed to query, filter, and sort data to ensure the correct data are selected for the particular analysis. The statistical analysis methods such as non-parametric tests are available in some spreadsheets and specialized statistical software packages, particularly those that render graphical outputs such as SAS, SPSS, R, R-Shiny, and the like.

Because audiometric data, employee (subject) data including calculated time or exposure intervals, and exposure classification (SEG) data are dynamic, the use of a real-time, automated information management software application designed to manage all aspects of collecting, aggregating, organizing, analyzing, interpreting and reporting audiometric data seamlessly in conjunction with employee and other related health and safety data and documentation is essentially necessary to practically utilize the invention. For this automation to be effective, it must be configured according to rules and logic specific to the business processes and particular organization, and deliver output (such as analysis and reports) in an accessible format.

Any sophisticated data management platform can be used to perform the calculations and analyses described herein. These include, but are not limited to environmental health and safety (EHS) platforms such as Cority, Gensuite, Cintellate, Enablon, Pure Safety, VelocityEHS, and others; audiometry-specific software or services such as Benson Solo, CounselEAR, Examinetics XM Solutions, HearTrak, Noah, Shoebox, Sycle, Workplace Integra and others; or occupational medicine or general medicine software platforms such as Agility, Allscripts, Epic, GalenMD, Meditech, OHM and others. In certain embodiments, the webOSCAR™ technology platform is utilized (URL www.webOSCAR.com). webOSCAR is a technology platform developed by an occupational medicine physician and designed to manage health and safety data (including but not limited to audiometric data) for highly regulated, hazardous industries.

*Other Applications of the Invention*

The application of this invention is not limited to detection and prediction of early NIHL. It can be readily adapted and modelled to evaluate hearing loss using applicable templates when such hearing loss is (1) characterized by a distinct or characteristic audiometric pattern in at least one phase of the disease process and (2) monitored for its progression by comparison of serial audiograms over time in a population of people. For example, templates can be developed to identify and predict moderate or advanced stages of hearing loss that develops after early NIHL has already developed. Though the focus of this disclosure is for prevention and screening for work-related hearing loss, particularly NIHL, the summary metric (W) and its derivatives ($\Delta dB_w$, $r_w$, $W_{L-R}$) can be constructed to perform a similar function for screening, detecting or predicting non-occupational hearing loss such as in motorcyclists, target shooters, or people who listen to music through devices such as mobile phones and earbuds.

The invention similarly can be applied to screen and predict other forms, causes or diseases of either sensorineural, conductive or mixed progressive hearing loss in any population, such as presbycusis in people over age 50, and otosclerosis in children by utilizing the $r_w$ in combination with the W and WTS to distinguish specific types of progressive hearing loss by their characteristic audiometric pattern. The invention could also be applied to people with any type of hearing disorder who wear hearing assistive devices (hearing aids) to measure improvement in functional hearing over time, and compare various types of devices. Finally, the invention could be applied in epidemiological studies to define what constitutes "normal" hearing levels or changes within a given population, such as school-age children or another demographic group.

The following examples are provided to describe the invention in greater detail. They are intended to illustrate, not to limit, the invention.

**Example 1: General Methods.**

*Data Collection and Scrubbing (Phase 1)*

Raw data from a Benson Solo audiometer software database were exported and compiled in an Excel spreadsheet. Employee-specific data (names) were de-identified and assigned sequential ID numbers. Demographic information in the database included date of birth, gender, test type (baseline, periodic or repeat), job title, and hearing protector type. The demographic data were scrubbed and, where applicable, corrected to ensure formatting consistency.

Similar exposure group (SEG) classifications based upon noise dosimetry measurements obtained by an industrial hygienist were categorized as follows:

- NIL: < 80 dB
- LOW: 80-85 dB
- MED: 85-90, 90-95 dB
- HIGH: 95-100, 100-105, 105-110 dB

Incomplete or invalid audiometric data (27 tests, from the original data set of 1,647 tests) were excluded from data analysis. Baseline and periodic test types were computed based upon the number of tests and time intervals between tests for each subject. STS values (-OSHA, - OSHA-REC, and -CSA) were calculated from the raw data.

The following metrics were computed in the webOSCAR™ platform using the '4kHzV1' template (previously described) to describe the typical audiometric pattern and magnitude of early NIHL (ENIHL) for an ear with 4 kHz nadir (peak hearing loss), as described above:

- W, the weighted hearing level, reported in $dB_W$.
- WTS, the Weighted Threshold Shift for periodic audiograms calculated as $\Delta dB_W$ (current test minus baseline test), reported in $dB_W$.
- $r_W$, the correlation coefficient that measures the extent of a linear relationship between a change in the current vs. baseline audiogram ($\Delta dB_W$) to the ENIHL pattern, calculated as values between -1.0 and +1.0. Based on the simulation study, a $r_W > 0.50$ is established as a threshold for ENIHL.
- $W_{L-R}$ (Laterality) which measures the extent of asymmetry between Left and Right ears' weighted hearing level (W, measured in $dB_W$) in an audiogram, reported in unitless values (>0).

Data were analyzed using a combination of SQL (Structured Query Language, Microsoft Corporation), SAS Version 9.4 software (SAS Institute, Cary, NC) and R 3.5.2 for Windows (https://www.r-project.org/). SQL and R are incorporated into the webOSCAR platform to automate the audiometric analytics.

*Computer Simulation (Phase 2A)*

This computer simulation measured the comparative sensitivity and specificity of the WTS ($\Delta dB_w$) and $r_w$ for identifying, predicting and measuring ENIHL in comparison to the STS (STS-OSHA and STS-CSA). Variant patterns and progressions of 'normal' hearing ("control" group), early NIHL (the 4kHzV1 template), and typical presbycusis were simulated at relatively low total levels of peak hearing loss with a nadir (maximum loss in hearing level) at 4 kHz. The receiver operating characteristics (sensitivity and specificity) were plotted. The results corroborated the $dB_w$ metrics have superior sensitivity and specificity for detecting and predicting early NIHL over the STS, particularly when the correlation to early NIHL ($r_w$) was applied.

*Statistical Analysis (Phase 2B)*

Individual employee audiometric data metrics were analyzed using Shewhart and cusum control charts (described above).

Aggregate analyses with the following *post hoc* non-parametric statistics were utilized on the W, WTS and derived metrics to measure and compare short- and long-term trends and changes of individuals and groups (within and among SEGs) within the employee population as explained above: Sen slope; Kruskal-Wallis and Mann-Whitney tests. Other non-parametric tests utilized for this analysis included the Spearman rank correlation coefficient (a correlation to test if two set of values are directly or inversely correlated); and Wilcoxon Signed rank test (to measure the magnitude of differences between two sets of data where the measurement scale is at least interval to test symmetry (up or down) around 0.0 values, wherein a low p value (<0.05) indicates absence of symmetry (i.e., presence of asymmetry) and a high value indicates presence of symmetry.

**Example 2: Demonstration** of Validity.

To demonstrate the invention works as intended, and in particular to demonstrate how the sensitivity and specificity of the Weighted Hearing Level (W) and Weighted Threshold Shift (WTS) compare to the STS for detecting individual audiometric progression toward early NIHL (ENIHL), predicting employees and SEGs at increased risk for NIHL, and objectively measuring aggregate trends within and among SEGs to assess the effectiveness of the HCP, a two-part research study was conducted in 2018-2019. In this Example, the metrics and statistical methodology described herein were applied to the collective audiometric database from a large gold mining company located in North America (the "Company") over the course of 6.25 years. A computerized simulation study with sensitivity and specificity analysis was also conducted to validate the methods.

Results are presented below. The employee demographics are summarized in Table 4. The subject population was predominantly comprised of males, and more than one quarter of subjects (27.2%) had at least one (1) baseline or periodic audiogram test at which the computed employee age is $\geq$ 50 years-the age threshold for increased risk for baseline or concomitant presbycusis, a potential confounder for STS determinations. Because employment start date was not captured, the duration of employment was not calculated.

**Table 4. Employee Demographics.**

| Metric | # | % | Comments |
|---|---|---|---|
| **Number of Employees** | 1067 | 100.0% | |
| Active | 1012 | | |
| Inactive | 55 | | |
| **Age @ Test** (Activity Date) | | | Numbers may not add to 100% due to overlap across age groups. |

| Metric | # | % | Comments |
|---|---|---|---|
| N = 1647 tests | | | |
| < 18 | 1 | 0.1% | |
| 18-29 | 359 | 21.8% | |
| 30-40 | 432 | 26.3% | |
| 41-50 | 393 | 23.9% | |
| 51-60 | 390 | 23.7% | |
| 61+ | 58 | 3.5% | |
| >50 | **448** | **27.2%** | At risk for presbycusis |
| Unknown | 12 | 0.7% | No Date of Birth |
| **Gender** | | | |
| Male | 893 | 83.7% | |
| Female | 155 | 14.5% | |
| Unidentified | 21 | 2.0% | |
| **Job Titles/Levels (SEG)** | | | Job Title/Level labels inconsistent or variable SEGs. |
| **Employment (years)** | | | Consecutive years In Hearing Conservation Program |
| 0 to <1 | NA | | |
| 1 to 3 | NA | | |
| 4 to 6 | NA | | |
| 7 to 9 | NA | | |
| 10 + | NA | | |
| NA = Not applicable or cannot be determined | | | |

Summarized in Table 5 is the test type distribution. Approximately two thirds (64.9%) of the 1,647 total valid audiogram tests conducted in the selected period were Baseline tests. Of the 1,067 employees, one third (33.3%) had only one (1) periodic test, 8.6% had two (2) consecutive periodic tests, and only 9 employees (0.8%) had three (3) consecutive tests. No (0) employees had four (4) or more consecutive tests. The mean (average) periodic test interval was 2.4 years, with approximately only one quarter (28.4%) occurring in the 'annual' interval range.

**Table 5. Distribution of Test Type (6.25-year time period).**

| Metric | # | % | Comments |
|---|---|---|---|
| **TESTS** | 1647 | 100.0% | |
| Removed from analysis | 27 | | Any reason including invalid or missing values |
| Repeat tests | 0 | | <50 days after previous test |
| Baselines | 1067 | 64.9% | Initial test |
| Periodic tests | 566 | 34.4% | Consecutive tests (not necessarily annual) |
| Prior STS (revised Baseline) | NA | | |
| **EMPLOYEES** | | | |
| Baseline only (1 Test) | 611 | 57.3% | |
| 1 Periodic Test | 355 | 33.3% | |
| 2 Periodic Tests | 92 | 8.6% | |
| 3 Periodic Tests | 9 | 0.8% | |
| 4 Periodic Tests | 0 | 0.0% | |
| 5+ Periodic Tests | 0 | 0.0% | |
| **PERIODIC TESTS INTERVALS (years)** | | | Periodic $\equiv$ >270 days for annual test |
| Average Interval | **2.4** | | N = 566 Periodic tests |
| 0.2-0.69 ("indeterminate") | 25 | 4.4% | Between 50 and 270 days apart |
| 0.7-1.3 ("on time") | **161** | **28.4%** | |
| >1.3-2.0 | 31 | 5.5% | |
| >2.0-3.0 | 219 | 38.7% | |
| >3.0 | 130 | 23.0% | |
| NA = Not applicable or cannot be determined | | | |

Tables 6A-6C summarize the statistics for the distribution of tests by exposure SEG, distribution of SEGs by job title and HPD assignment, and distribution of tests by hearing protection and HPD type. Over half (60.5%) of tests are reported in employees in MED and HIGH SEGs (*see* Table 6A). Consistent recording of Job Title or Level by SEG was not available. As shown in Table 6B, data on HPD usage by Employee and Job Title/Level were not systematically recorded. Moreover, nearly two thirds (61.0%) of tests are reported with associated use of hearing protection devices (*see* Table 6C).

**Table 6A. Distribution of Tests by Exposure SEG.**

| EXPOSURE SEG (N=1645 tests) | Tests | % | # Baseline | % | # Periodic | % |
|---|---|---|---|---|---|---|
| **NIL** (< 80 dB) | 367 | 22.3% | NA | | NA | |
| **LOW** (80-85 dB) | 279 | 16.9% | NA | | NA | |
| **MED** (85-90, 90-95 dB) | **655** | **39.8%** | NA | | NA | |
| **HIGH** (95-100, 100-105, 105-110 dB) | **341** | **20.7%** | NA | | NA | |
| **Indeterminate** | 3 | 0.3% | NA | | NA | |
| NA = Not applicable or cannot be determined | | | | | | |

### Table 6B. Distribution of SEGs by Job Title and Hearing Protection Assignment

| EXPOSURE SEG (N=1645 tests) | Job Titles in SEG | Tests, HP | % |
|---|---|---|---|
| **NIL** (< 80 dB) | NA | NA | |
| **LOW** (80-85 dB) | NA | NA | |
| **MED** (85-90, 90-95 dB) | NA | NA | |
| **HIGH** (95-100, 100-105, 105-110 dB) | NA | NA | |
| **Indeterminate** | NA | NA | |
| HP = Hearing protection (assigned, any type) NA = Not applicable or cannot be determined | | | |

### Table 6C. Distribution of Tests by Hearing Protection and HP Type.

| HEARING PROTECTION DEVICE (HPD) TYPE (N=1645 tests) | Tests | % |
|---|---|---|
| **Tests without HPD** | 640 | 38.9% |
| **Tests with HPD** | 1005 | 61.0% |
| **Custom made ear plugs** | 12 | |
| **Muff/Plugs** | 414 | |
| **Muffs** | 153 | |
| **Piston (plugs)** | 1 | |
| **Plugs** | 425 | |

In Table 7, the statistics for the distribution of baseline tests was summarized. Over half (60.1%) of baseline audiograms were 'abnormal' insofar as having a hearing loss of ≥25 dB in at least one frequency in either ear. Further, nearly one third (31.8%) of such abnormalities demonstrated moderate severity (40-50 dB) and nearly one fifth (18.4%) were severe. Of all these abnormal baseline tests, none (0.0%) demonstrated a conductive hearing loss pattern (e.g., middle ear damage or cerumen impaction). One quarter (25.1%) demonstrated a peak hearing loss at 4 kHz, less than half (10.5%) of which had concomitant loss at 8 kHz, and nearly that same proportion (13.1%-21.4%) had a "downsloping" pattern. Collectively these findings suggested that as many as 15-25% of baselines represented a pre-existing NIHL.

### Table 7. Distribution of Baseline Tests as Normal or Abnormal.

| BASELINE TESTS | # | % | Comments |
|---|---|---|---|
| **Total baseline tests** | 1067 | 100.0% | 1067 tests x 2 ears = 2134 |
| **≥1 hearing level (Li) at ANY frequency =** | | | Either or both ears |
| 25-35 dB | 641 | 60.1% | |
| 40-50 dB | 339 | 31.8% | |
| ≥ 55 dB | 196 | 18.4% | |
| **Hearing level (Li) at 4 kHz =** | | | Either or both ears |
| 25-35 dB | 268 | 25.1% | |
| 40-50 dB | 155 | 14.5% | |
| ≥ 55 dB | 119 | 11.2% | |
| **Hearing level (Li) at 4 kHz AND 8 kHz =** | | | Either or both ears |
| 25-35 dB | 112 | 10.5% | |
| 40-50 dB | 57 | 5.3% | |
| ≥ 55 dB | 74 | 6.9% | |
| **ALL hearing levels =** | | | ≡ "conductive hearing loss" either or both ears |
| 25-35 dB | 0 | 0.0% | |

(continued)

| BASELINE TESTS | # | % | Comments |
|---|---|---|---|
| 40-50 dB | 0 | 0.0% | |
| ≥ 55 dB | 0 | 0.0% | |
| AVG (4,6,8 kHz) - AVG (0.5,1,2 kHz) = | | | Either or both ears. 'Downsloping' pattern |
| 10-19 dB | 228 | 21.4% | |
| 20-29 dB | 106 | 9.9% | |
| ≥30 dB | 124 | 11.6% | |
| AVG (2,3,4 kHz) - AVG (0.5,1,2 kHz) = | | | Either or both ears. 'Downsloping' pattern |
| 10-19 dB | 140 | 13.1% | |
| 20-29 dB | 75 | 7.0% | |
| ≥30 dB | 32 | 3.0% | |
| Hearing loss classifications: 25-35 dB = "mild" 40-50 dB = "moderate" ≥55 dB = 'moderately severe'. These categories may have overlapping records. Example: a test may have one frequency in the 25-35 dB range and another frequency in the 40-50 dB range. Thus, the sums exceed the total count for the baseline tests, and the sums of the percentages will be > 100%. | | | |

The STS outcomes are summarized in Table 8. A total of 117 audiograms (7.1% of periodic tests) had an STS (-OSHA or -CSA) in either ear, but only a small subset (12 audiograms, 0.7% of periodic tests) had bilateral STS. The distribution between right (64) and left (65) ears was equal. In addition, the total number of periodic audiograms that met the STS-OSHA criteria (which in contrast to STS-CSA does not include large individual high frequency changes) was 61 (3.7% of periodic tests) in either ear, with a similar low subset (6 audiograms, 0.4% of all periodic tests) of bilateral STS. When OSHA recordability criteria were applied, the total number of periodic audiograms that met the STS-OSHA-REC criteria was 40 (2.4% of periodic tests). Approximately 25% of periodic audiograms meeting the STS-OSHA definition thus occurred with relatively low absolute hearing levels in the higher (2, 3, and 4 kHz) frequencies.

**Table 8. Distribution of Periodic Test Computed STS Outcomes by Exposure.**

| Metric | Tests | %, N=566 tests |
|---|---|---|
| STS-CSA [Δ234 Avg >= 10 dB OR (ΔL3KHz >= 15 dB OR ΔL4KHz >= 15 dB] | | |
| STS-CSA-L Ear | 64 | 3.9% |
| STS-CSA-R Ear | 65 | 4.0% |
| STS-CSA-EITHER Ear | 117 | 7.1% |
| STS-CSA-BOTH Ears | 12 | 0.7% |
| STS-OSHA [Δ234 Avg >= 10 dB] | | |
| STS-OSHA-L Ear | 33 | 2.0% |
| STS-OSHA-R Ear | 34 | 2.1% |
| STS-OSHA-EITHER Ear | 61 | 3.7% |
| STS-OSHA-BOTH Ears | 6 | 0.4% |
| STS-REC-OSHA [Δ234 Avg >= 10 dB AND 234 Avg (Current test) >= 25 dB] | | |
| STS-REC-OSHA-L Ear | 24 | 1.5% |
| STS-REC-OSHA-R Ear | 20 | 1.2% |
| STS-REC-OSHA-EITHER Ear | 40 | 2.4% |
| STS-REC-OSHA-BOTH Ears | 4 | 0.2% |

*Individual Analytics (Comparative Snapshot & Trends)*

For employees ('subjects') with at least one (1) periodic test plus Baseline test (minimum 2 tests total), control charts were generated to plot individual $dB_W$ levels from test-to-test, in comparison to 'control' data (which for this project is derived from the 'Nil' SEG). These charts had the most value for employees with a higher number of consecutive periodic tests, as illustrated for Employee #1821 with the maximum number of periodic tests (3). As shown in Table 9, Employee 1821 exhibited a moderate progressive increase in $\Delta dB_W$ in the LEFT ear indicating early NIHL, but no corresponding change in the RIGHT ear. For instance, the LEFT ear Correlations ($r_W$) between the three (3) post-baseline audiograms and the NIHL template were 0.22, 0.3 and 0.63, respectively, which is consistent with developing progressive NIHL in the left ear. As the hearing loss increased, both the WTS ($\Delta dB_W$) and the $r_W$ values increased such that by post-baseline Test #2, the WTS ($\Delta dB_W$ = 11.9) crossed the 10 $dB_W$ threshold to *flag* as *possible* NIHL. By the third post-baseline observation, the WTS ($\Delta dB_W$ = 20.27) and the $r_W$ (0.63, >> 0.5 threshold) were sufficiently high to confirm with high probability that this ear had sustained ENIHL-which the lagging STS indicators did not detect until Test #3. The Shewhart and Cusum charts illustrate these progressions (*see* Figures 3A and 3B, respectively). Thus, the combination of WTS and $r_W$ predicted ENIHL nearly two years before the lagging STS values (OSHA and CSA) reached the 10 dB threshold.

In comparison, in the RIGHT ear correlation ($r_W$) values were negative or hovered around 0.0, and the WTS ($\Delta dB_W$) values decreased over time. These findings suggested that minimal hearing loss progression that did not follow an ENIHL pattern. The gradually increasing Laterality described this asymmetry in hearing loss between ears.

## Table 9. Employee #1821 Audiometric Raw and Derived Data

### 9A. Demographics.

- DOB (Date of and Gender: 6/27/1964; Male
- # Tests: 4 (Baseline + 3 Periodic Tests)
- Duration: 5.49 years
- Exposure Group(s): High, then Medium

| Test Age | Job Title | Department | Exposure | PPE | Protector Type | Activity Date | Test Interval (yrs) | Test Type |
|---|---|---|---|---|---|---|---|---|
| 49 | Miner 2 | Underground | 95 - 100 dB | TRUE | Plugs | 7/25/2013 | | Baseline |
| 51 | Miner 2 | Underground | 95 - 100 dB | TRUE | Plugs | 8/7/2015 | 2.04 | Periodic |
| 52 | Miner 4 | Underground | 95 - 100 dB | TRUE | Plugs | 2/3/2017 | 1.50 | Periodic |
| 54 | Miner 4 | Underground | 85 - 90 dB | TRUE | Muff/Plugs | 1/15/2019 | 1.95 | Periodic |

### 9B. Raw audiometric results.

| Test # | Left 0.5K | Left 1K | Left 2K | Left 3K | Left 4K | Left 6K | Left 8K | Right 0.5K | Right 1K | Right 2K | Right 3K | Right 4K | Right 6K | Right 8K |
|---|---|---|---|---|---|---|---|---|---|---|---|---|---|---|
| B | 5 | 5 | 35 | 20 | 10 | 20 | 5 | 0 | 5 | 5 | 5 | 10 | 20 | 10 |
| 1 | 5 | 5 | 35 | 30 | 10 | 40 | 20 | 5 | 0 | 0 | 5 | 5 | 20 | 10 |
| 2 | 5 | 5 | 35 | 25 | 15 | 40 | 20 | 5 | 5 | 5 | 5 | 15 | 15 | 20 |
| 3 | 5 | 5 | 35 | 30 | 30 | 25 | 30 | 5 | 5 | 5 | 5 | 10 | 15 | 20 |

## 9C. STS determinations.

| Test # | 234AvgL | Δ234AvgL (C-B) | 234AvgR | Δ234AvgR (C-B) | STS-CSA | STS-OSHA | STS-OSHA-REC |
|---|---|---|---|---|---|---|---|
| B | 21.7 | | 6.7 | | | | |
| 1 | 25.0 | 3.3 | 3.3 | -3.4 | No | No | No |
| 2 | 25.0 | 3.3 | 8.3 | 1.6 | No | No | No |
| 3 | 31.7 | **10.0** | 6.7 | 0.0 | **Left** | **Left** | **Left** |

## 9D Metrics: $dB_w$, $\Delta dB_w$, $r_w$ and $W_{L-R}$

| Test # | dB L | ΔdB L (C-B) | r L | dB R | ΔdB R (C-B) | r R | $W_{L-R}$ (no units) |
|---|---|---|---|---|---|---|---|
| B | 13.6 | | | 13.8 | | | 0.0 |
| 1 | 23.5 | 9.9 | 0.22 | 10.9 | -2.9 | -0.21 | 0.7 |
| 2 | 25.5 | **11.9** | 0.38 | 16.1 | 2.3 | 0.02 | 0.5 |
| 3 | 33.8 | **20.2** | **0.63** | 12.6 | -1.2 | -0.30 | 0.9 |

*'You are Here' Charts*

For employees with at least two consecutive periodic tests plus Baseline test (3 tests total), "You are Here" distributions were generated that indicated the individual's ENIHL metrics in comparison to peers (which for this project is derived from the entire study population with one or more periodic test). The charts depicted in Figures 4-6 had the most value for Employees with a higher number of consecutive periodic tests, as illustrated for Employee #1821 with the maximum number of periodic tests (3). For the left ear, the $dB_w$ and annualized trend in $dB_w$ increased (*see* Figure 4, panel A and Figure 5, panel A) at a rate of approximately 3 $dB_w$ per year toward ENIHL, which was in the upper range of distribution in audiometric changes compared with other employees across the organization. For the right ear, the $\Delta dB_w$ remained near 0 (*see* Figure 4, panel B and Figure 5, panel B) and neither progressed year-to-year nor correlated with ENIHL (*see* Figure 6, panel B), and thus was similar to the distribution of most other employees across the organization. The asymmetry between left and right ear changes toward NIHL suggested possible non-occupational etiologies or risk factors for NIHL (*e.g.,* shooting), which suggested further query was needed.

*Aggregate Analytics (HCP Effectiveness)*

The aggregate comparative trends toward ENIHL across exposure groups was studied. Figure 7 displays box plots of comparative trends toward ENIHL, calculated as pooled $\Delta dB_w$/year (current vs. baseline test), by SEG for all employees with ≥2 tests (N = 456 total) for left and right ears. Negative values indicated annual declines or stability in $dB_w$. On the other hand, positive values indicated annual increases in $dB_w$/time. For Employees who moved from one SEG to another and had values in more than one SEG, the initial SEG was assigned for this analysis. Further, only high outliers were relevant for interpretation of audiometric data because hearing generally tends to worsen or stay the same, but not improve, over time. The outlier values in this non-parametric test were determined by rank, not confidence intervals, and the comparisons were without regard to the correlationΔ values. The relatively small number of longitudinal test data limited the long-term interpretability of the data set.

As can be seen in Figure 7, no statistically significant, aggregate trends in $\Delta dB_w$/year among the four (4) SEGs were detected. The median values were all close to 0. This finding indicated that for the given data set, the risk of ENIHL in the higher (MED and HIGH) exposed groups (with or without hearing protection) was the same as those with LOW or NIL exposure groups (with or without hearing protection). The overall effectiveness of the HCP for this time period was therefore interpreted as high. All SEGs showed a slight upward (positive) annualized $dB_w$ trend, which was an expected finding given that hearing tends to worsen or stay unchanged rather than improve over time. A relatively small number of high outliers for $\Delta dB_w$/year were identified for the MED and HIGH SEGs-an expected finding which suggested that only a few tests were outside (above) the control range. These high outliers warranted individual investigation to determine if in fact hearing protection was being utilized correctly. A Kruskall-Wallis one-way ANOVA (2-tailed) of $\Delta dB_w$ demonstrated no difference among the median values of the $\Delta dB_w$/year values for the four (4) SEGs for Left ear (p = 0.10) and Right ear (p = 0.64). All median values of $\Delta dB_W$ were close to 0. Statistically significant asymmetry toward increased hearing loss was present by signed rank test in all SEGs (see Table 10).

**Table 10. Kruskall-Wallis test of $\Delta dB_W$--medians, 95th percentile and asymmetry**

| | Left Ear (p = 0.10) | | | | Right Ear (p = 0.64) | | | |
|---|---|---|---|---|---|---|---|---|
| | Nil | Low | Med. | High | Nil | Low | Med. | High |
| **Median** | 3.78 | 3.56 | 3.35 | 3.38 | 1.27 | 3.31 | 3.45 | 1.94 |
| **95%** | 19.41 | 14.10 | 15.05 | 14.52 | 12.02 | 13.46 | 12.61 | 13.51 |
| **Signed rank, p** | <0.0001 | <0.0001 | <0.0001 | <0.0001 | 0.0133 | <0.0001 | <0.0001 | 0.0014 |

Shown in Figure 8 are box plots showing test-to-test annualized trends toward ENIHL (pooled Sen slopes). For employees who moved from one SEG to another and had values in more than one SEG, the initial SEG was assigned for this analysis. The outlier values in this non-parametric test were determined by rank, not confidence intervals, and the comparisons were without regard to the correlation$\Delta$ values. The Kruskall-Wallis one-way ANOVA statistical analysis is summarized in Table 11. As can be seen in Figure 8 and Table 11, there was no difference among the median values of the Sen slopes for the four (4) SEGs for Left ear (p = 0.67) and Right ear (p =0.11). This finding suggests that the progression toward early NIHL in the higher exposure SEGS (with or without hearing protection) was the same as those with LOW or NIL exposure (with or without hearing protection). A relatively small number of high outliers for $\Delta dB_W$ /year were identified for the MED and HIGH SEGs-an expected finding which suggested a few tests were outside the control range. The Sen slopes tended to be slightly positive as a group ($\leq 1$ $dB_W$/year), reflected by significant signed rank tests, across all SEGs-an expected finding given that hearing tends to worsen or stay unchanged rather than improve over time.

**Table 11. Kruskall-Wallis test of pooled Sen slopes-medians, 95th percentile and asymmetry.**

| | Left Ear (p = 0.67) | | | | Right Ear (p = 0.11) | | | |
|---|---|---|---|---|---|---|---|---|
| | Nil | Low | Med. | High | Nil | Low | Med. | High |
| **Median** | 1.284 | 1.650 | 1.047 | 1.178 | 0.837 | 1.197 | 0.712 | 0.558 |
| **95%** | 5.276 | 5.586 | 5.568 | 5.374 | 4.430 | 5.308 | 4.544 | 5.352 |
| **Signed rank, p** | <0.0001 | <0.0001 | <0.0001 | <0.0001 | 0.0089 | <0.0001 | 0.0024 | 0.0003 |

Shown in Figure 9 are box plots showing Weighted Correlation Coefficients ($r_w$) for changes in the audiometric pattern from baseline toward the expected pattern of early NIHL, by SEG. For employees who moved from one SEG to another and had values in more than one SEG, the initial SEG was assigned for this analysis. The outlier values in this non-parametric test were determined by rank, not confidence intervals, and comparisons were without regard to the $\Delta dB_W$ values. The Kruskall-Wallis one-way ANOVA statistical analysis is summarized in Table 12. According to Figure 9 and Table 12, no statistically significant correlations of change specifically toward ENIHL were *detected* among the four (4) SEGs. This finding suggested that audiograms in the higher exposed SEGs were generally not progressing specifically toward the pattern expected for early NIHL compared with the lower exposed SEGs. Moreover, a statistically significant asymmetry toward increased hearing loss was present by signed rank test in all SEGs except right ear NIL (see Table 12).

**Table 12. Kruskall-Wallis test of correlation to early NIHL-- medians, percentiles and asymmetry.**

| | Left Ear (p = 0.65) | | | | Right Ear (p = 0.49) | | | |
|---|---|---|---|---|---|---|---|---|
| | Nil | Low | Med. | High | Nil | Low | Med. | High |
| **Mean** | 0.16 | 0.19 | 0.13 | 0.16 | 0.01 | 0.14 | 0.10 | 0.06 |
| **Std Deviation** | 0.40 | 0.36 | 0.41 | 0.41 | 0.42 | 0.39 | 0.40 | 0.39 |
| **25 %ile** | -0.13 | -0.02 | -0.14 | -0.12 | -0.36 | -0.13 | -0.21 | -0.21 |
| **Median** | 0.19 | 0.16 | 0.18 | 0.22 | -0.02 | 0.18 | 0.11 | 0.22 |
| **75%ile** | 0.48 | 0.44 | 0.44 | 0.46 | 0.36 | 0.41 | 0.37 | 0.38 |
| **95 %ile** | 0.85 | 0.76 | 0.75 | 0.78 | 0.70 | 0.80 | 0.79 | 0.78 |

(continued)

| | Left Ear (p = 0.65) | | | | Right Ear (p = 0.49) | | | |
|---|---|---|---|---|---|---|---|---|
| | Nil | Low | Med. | High | Nil | Low | Med. | High |
| **Signed rank, *p*** | 0.0003 | <0.0001 | <0.0001 | <0.0001 | 0.8907 | 0.0003 | 0.0004 | 0.0818 |

*Distributions of Trends toward Early NIHL*

Shown in Figure 10 are frequency distribution (density) plots displaying the WTS ($\Delta dB_w$) for all employees' most recent periodic test. In both left and right ears, a majority of periodic tests had very minimal positive change in median $dB_w$ (Left = +2.5 $dB_w$; Right median = +1.5 $dB_w$). These values indicated that most periodic audiograms have remained essentially unchanged in terms of progression toward ENIHL, and that there was little asymmetry in the distribution between Left and Right ears. In a theoretically noise "unexposed" population, with no trend toward NIHL, median $\Delta dB_w$ should be 0 in either ear. However, differential (right vs. left) ear $dB_w$ values would still be expected. It is important to note the underlying distribution is continuous, and histograms artificially put data into discrete bins which may or may not be uniform. The smoothed curves on the graphs shown in Figure 10 are kernel density estimates which remove discontinuity within the edge of each bin. The little tail to the left of 0 is an artifact. The process of smoothing tends to increase the spread of the distribution slightly relative to the histogram.

Figure 11 displays the frequency distribution of the Sen slopes for all employees' most recent periodic tests. In both left and right ears, most of the periodic tests had very minimal rate of change in $dB_w$ from test to test (Right median = 0.0; Left median = 1.25). These values indicated that most periodic audiograms remained unchanged in terms of progression toward ENIHL, and that there was not asymmetry in the distribution between Left and Right. As with $\Delta dB_w$, the median Sen slope is 0 with no trend toward NIHL in theoretically "unexposed" populations. Further, a wide range of correlation values was present in both the left and right ears *(see* Figure 12). Correlations greater than 0.50 corresponded to increased probability of ENIHL, but represented to a small fraction of all periodic test results.

*Symmetry of Changes toward Early NIHL*

Shown in Figure 13 is a frequency distribution (density plot) of the difference of the left ear and the right ear for all employees' most recent periodic tests. The median difference in $\Delta dB_w$ between ears (a measure of asymmetry) was close to 0.0, indicating minimal asymmetric progression toward early NIHL among all periodic audiograms. Additionally, the extent of severe asymmetry in a small proportion of tests was reflected in the width of the histogram. Moreover, systematic differences between the right and left ears were not expected with an occupationally exposed population.

Scatter plot analysis between left and right ears was also performed on the employee population for their most recent periodic tests. As shown in Figures 14 and 15, most Sen slopes (annualized trend in $dB_w$) of employees' most recent periodic tests clustered around "no change" for both left and right ears, with very few highly asymmetrical outliers. A small fraction of periodic audiogram results demonstrated bilateral significant changes compared with STS criteria. These latter tests can be flagged as potential outliers, though they may also be early indications of bilateral NIHL. A Spearman correlation of the left vs right ear for $\Delta dB_w$ was 0.229 (*p* < 0.0001). For the Sen slopes, the Spearman correlation was 0.183 (*p* < 0.0001). Both of these correlations were low (operating independently of one another), accounting for < 5% of the subject-to-subject variability but nonetheless were statistically significant. Further, the outliers indicated that these responses were not typical of the group as a whole.

*Suspected Early NIHL Outlier Detection*

Shown in Figure 16 are scatter plots of $\Delta dB_w$ and corresponding correlation of change toward early NIHL in the left ear and right ear for ALL employees' most recent periodic test. A small fraction of periodic audiogram results demonstrated both a specific ENIHL pattern and a significant change toward NIHL. These tests may be identified as either detections of ENIHL (true positives) or else outliers due to other factors (false positives). Since outliers can be real (true positives) or erroneous (false positives), all subjects with a positive outlier test can be followed up to determine the clinical significance of the result.

This may include additional testing, verification that the subjects are making appropriate use of protective headgear, or in the extreme, transfer out of high-exposure environments. Further, by setting the $\Delta dB_w$ criteria lower (*e.g.,* to 10 $dB_w$ to correspond to STS thresholds), the sensitivity (*i.e.,* more early true positives) increased with the risk of decreasing specificity (*i.e.,* more early false positives).

*Summary*

The Company audiometric database contained a significant amount of valuable audiometric data that, upon robust analysis as described above, allowed important statistical findings to be made and conclusions to be reached at an aggregate and individual employee level. Based upon the foregoing data analysis, the Company's HCP appeared to be effective at preventing early NIHL, particularly among employees with moderate and high measured external noise exposures. More definitive conclusions about audiometric trends and HCP effectiveness, and more specific identification and handling of outliers to verify early NIHL cases, would benefit from a larger number of longitudinal audiograms (longer duration) and more consistent collection of periodic (annual) tests at recurring frequency intervals. With such consistently collected longitudinal data and the right platform to schedule, collect, analyze, and report them, these metrics and statistical methods can be readily incorporated into an Audiometric Best Practices to effectively manage noise-related risk.

In conjunction with existing on-site audiometric testing and EHS systems, these Audiometric Best Practices methods and technology can be implemented at all operations across the entire Company as a standard operating procedure. This approach would substantially improve corporate-wide program performance and outcomes related to measuring and controlling risk for occupational noise exposure, and would directly benefit and protect the many workers who wear hearing protection to prevent NIHL.

Various modifications of the invention, in addition to those described herein, will be apparent to those skilled in the art from the foregoing description. Such modifications are also intended to fall within the scope of the appended claims.

**Claims**

1. A method for detecting a change in a hearing loss condition, comprising the steps of:

   identifying the hearing loss condition measured in at least one pure-tone audiogram by a characteristic pattern and a relative magnitude in hearing levels measured in an ear in at least one of a sound frequency, a range of frequencies, or in a combination of frequencies, in comparison with one or more hearing levels measured at a plurality of other frequencies in the audiogram;

   providing an expected hearing response function, $f_i$, for the hearing loss condition calculated from a plurality of sound frequencies, i, measured in the audiogram of the ear, wherein $f_1$ is a scalar, which includes a unitless proportional value, assigned for the hearing level;

   generating a weighted hearing level, W, from a measured hearing level associated with the subject for each ear from the audiogram and the expected hearing response function ($f_i$) for the hearing loss condition according to a first equation:

$$\mathbf{W} = \bar{L} + \frac{\Sigma_i L_i (f_i - \bar{f})}{\Sigma_i (f_i - \bar{f})^2} (f_m - \bar{f})$$

   wherein

   $W$ is the weighted hearing level that summarizes correspondence between the measured audiogram and the hearing loss condition in the ear, reported as a numerical value in decibel, dB, -equivalent units termed 'dBw';

   $i$ are the sound frequencies for which a hearing response is expected and a hearing level was measured in the audiogram of the ear;

   $L_i$ is the measured hearing level for each sound frequency ($i$) in the ear as recorded in the audiogram associated with the subject, measured in dB;

   $\bar{L}$ is a first arithmetic mean of the measured hearing levels $L_i$, calculated in dB;

   $f_i$ is the expected hearing response function at each recorded sound frequency ($i$);

   $\bar{f}$ is a second arithmetic mean of the $f_i$ values;

   $\Sigma_i$ represents a summation over the i values; and

   $f_m$ is the maximum value in the set of $f_i$ values;

   calculating W for a baseline audiogram and calculating W for a subsequent audiogram for each ear of the subject, using the same expected hearing response function ($f_i$);

   calculating a ∆W for each ear of the subject by subtracting the W value calculated from the baseline audiogram from the W value calculated from the subsequent audiogram;

   generating a weighted correlation coefficient, $r_W$, from the measured hearing level ($L_i$) and the expected

hearing response function ($f_i$) for each ear from the baseline audiogram and the subsequent audiogram of the subject, according to a second equation:

$$\mathbf{r_W} = \frac{\Sigma_i((L_{it} - L_{i0}) - (\bar{L}_t - \bar{L}_0))(f_i - \bar{f})}{\sqrt{\Sigma_i(f_i - \bar{f})^2 \Sigma_i((L_{it} - L_{i0}) - (\bar{L}_t - \bar{L}_0))^2}}$$

wherein

$r_W$ is a statistical correlation metric measuring the extent of a linear relationship between a change in hearing toward the hearing loss condition from the baseline audiogram to the subsequent audiogram in the ear of the subject based on the expected hearing response function ($f_i$);
0 is a first subscript that denotes the baseline audiogram;
$t$ is a second subscript that denotes the subsequent audiogram; and
the remainder of the symbols are the same as those defined in the first equation;
determining if the $\Delta$W value and the corresponding $r_W$ value for each ear in the subject indicates that the hearing loss condition has improved, worsened, or remained unchanged at the time interval between the baseline audiogram and the subsequent audiogram; and,
estimating a trend in hearing toward the hearing loss condition by calculating a $\Delta$W per unit time in each ear of the subject between the subsequent audiogram and the baseline audiogram, expressed as an absolute rate of change in W per year, a $\Delta$W/year, and a proportional rate of change in W per year.

2. The method of claim 1, wherein the hearing loss condition is an early audiometric phase of noise-induced hearing loss (NIHL).

3. The method of claim 2, wherein the $f_i$ weightings comprise a template of the expected hearing response in the ear for early audiometric phase NIHL, selected from the templates consisting of:

0.5 kHz given 0.0 weight, 1 kHz given 0.0 weight, 2 kHz given 0.0 weight, 3 kHz given a weight of 1.0, 4 kHz given a weight of 2.0, 6 kHz given a weight of 1.0 and 8 kHz given a weight of 0.5;
0.5 kHz given 0.0 weight, 1 kHz given 0.0 weight, 2 kHz given 0.0 weight, 3 kHz given a weight of 0.5, 4 kHz given a weight of 2.0, 6 kHz given a weight of 1.0 and 8 kHz given a weight of 0.5; and
0.5 kHz given 0.0 weight, 1 kHz given 0.0 weight, 2 kHz given 0.0 weight, 3 kHz given a weight of 1.0, 4 kHz given a weight of 2.0, 6 kHz given a weight of 0.5 and 8 kHz given a weight of 0.5.

4. The method of claim 2, wherein the $\Delta$W value of at least 10 dBw indicates a shift toward early NIHL in the ear of the subject.

5. The method of claim 1, comprising utilizing the measured hearing level data for the subject from the baseline audiogram and a plurality of subsequent audiograms conducted over a plurality of subsequent time intervals.

6. The method of claim 1, further comprising transforming the measured hearing level data from the plurality of pure-tone audiograms associated with the subject and the expected hearing response function ($f_i$) for the hearing loss condition into the weighted hearing level associated with the subject for each ear, to detect an aggregated change over time for at least one of a population and a sub-population, the method further comprising the steps of:

having a plurality of subjects assigned to at least one of a defined population and a defined sub-population, in which each subject has a plurality of audiograms that includes the baseline audiogram which is administered to the subject upon entering the population, and at least the subsequent audiogram;
applying a $r_W$ cutoff criterion value to each corresponding $\Delta$W value for each ear in each audiogram among the subjects in the population to exclude the subsequent audiogram with the $\Delta$W value that does not positively correlate with the hearing loss condition from the measurements of the aggregated population changes over time;
calculating the mean average or median value of the $\Delta$W/year trend for each ear including the baseline audiogram and the subsequent audiograms, for each subject in the population over a period of time;
calculating at least one of an arithmetic mean and a median of the $\Delta$W/year trend values for each subject for each ear, for a population $\Delta$W/year trend value;
calculating the mean average or median value of the population $\Delta$W/year trend value for each subject for each ear in the population;
applying at least one statistical method to do at least one of measuring and comparing trends for hearing loss metrics that relate to the hearing loss condition for at least one of the population and the sub-population.

7. The method of claim 6, wherein the hearing loss condition is an early audiometric phase of NIHL.

8. The method of claim 7, wherein the $f_i$ weightings comprise a template of the expected hearing response in the ear for early audiometric phase NIHL, selected from the templates consisting of:

   0.5 kHz given 0.0 weight, 1 kHz given 0.0 weight, 2 kHz given 0.0 weight, 3 kHz given a weight of 1.0, 4 kHz given a weight of 2.0, 6 kHz given a weight of 1.0 and 8 kHz given a weight of 0.5;
   0.5 kHz given 0.0 weight, 1 kHz given 0.0 weight, 2 kHz given 0.0 weight, 3 kHz given a weight of 0.5, 4 kHz given a weight of 2.0, 6 kHz given a weight of 1.0 and 8 kHz given a weight of 0.5; and
   0.5 kHz given 0.0 weight, 1 kHz given 0.0 weight, 2 kHz given 0.0 weight, 3 kHz given a weight of 1.0, 4 kHz given a weight of 2.0, 6 kHz given a weight of 0.5 and 8 kHz given a weight of 0.5.

9. The method of claim 7 wherein the trends for hearing loss that relate to the hearing loss condition for at least one of the population and the sub-population are applied to evaluate the effectiveness of a hearing conservation program for preventing NIHL in a least one of the population, the populations, and the sub-populations.

10. The method claim 7 wherein the trends for hearing loss that relate to the hearing loss condition for at least one of the population and the sub-population are applied to identify at least one of the subjects, the sub-populations, the population, and the populations at increased risk for development of NIHL.

11. The method of claim 7 wherein the trends for hearing loss that relate to the hearing loss condition for at least one of the population and the sub-population are applied to measure the effectiveness of a preventive method for noise exposure control within at least one of the population, the populations, and the sub-populations.

12. A system comprising at least one processor and a non-transitory computer readable medium comprising instructions that, when executed by the at least one processor, cause the system to perform the method of any one of claims 1-11.

13. A non-transitory computer readable medium comprising instructions that, when executed by at least one processor, cause the at least one processor to perform the method of any one of claims 1-11.

**Patentansprüche**

1. Verfahren zum Detektieren einer Änderung in einem Hörverlustzustand, das folgende Schritte umfasst:

   Identifizieren des Hörverlustzustands, der in zumindest einem Reintonaudiogramm durch ein charakteristisches Muster gemessen wurde, und einer relativen Höhe von Hörpegeln, gemessen in einem Ohr in zumindest einem aus einer Tonfrequenz, einem Frequenzbereich oder einer Kombination von Frequenzen, im Vergleich zu einem oder mehreren Hörpegeln, die bei einer Vielzahl von anderen Frequenzen im Audiogramm gemessen wurden;
   Bereitstellen einer erwarteten Hörreaktionsfunktion, $f_i$, für den Hörverlustzustand, berechnet aus einer Vielzahl von Tonfrequenzen, i, die im Audiogramm des Ohrs gemessen wurden,
   wobei $f_i$ ein Skalar ist, der einen einheitslosen proportionalen Wert umfasst, der dem Hörpegel zugewiesen ist;
   Erzeugen eines gewichteten Hörpegels, W, aus einem gemessenen Hörpegel, der dem Individuum für jedes Ohr aus dem Audiogramm zugeordnet ist, und der erwarteten Hörreaktionsfunktion $(f_i)$ für den Hörverlustzustand gemäß einer ersten Gleichung:

$$\mathbf{W} = \bar{L} + \frac{\Sigma_i L_i (f_i - \bar{f})}{\Sigma_i (f_i - \bar{f})^2}(f_m - \bar{f})$$

   worin

   $W$ der gewichtete Hörpegel ist, der die Entsprechung zwischen dem gemessenen Audiogramm und dem Hörverlustzustand im Ohr zusammenfasst, verzeichnet als Zahlenwert in Dezibel-, dB-, äquivalenten Einheiten, die als "dBw" bezeichnet werden,

$i$ die Tonfrequenzen sind, für die eine Hörreaktion erwartet wird und ein Hörpegel im Audiogramm des Ohrs gemessen wurde;

$Li$ der gemessene Hörpegel für jede Tonfrequenz $(i)$ im Ohr, wie in dem dem Individuum zugeordneten Audiogramm aufgezeichnet, gemessen in dB, ist;

$\bar{L}$ ein erstes arithmetisches Mittel der gemessenen Hörpegel $Li$, berechnet in dB, ist;

$fi$ die erwartete Hörreaktionsfunktion bei jeder aufgezeichneten Tonfrequenz $(i)$ ist;

$\bar{f}$ ein zweites arithmetisches Mittel der $fi$-Werte ist;

$\Sigma_i$ eine Summierung über die $i$-Werte ist; und

$f_m$ der Maximalwert im Satz von $fi$-Werten ist;

Berechnen von $W$ als Basislinien-Audiogramm und Berechnen von $W$ für ein darauffolgendes Audiogramm für jedes Ohr des Individuums unter Verwendung der gleichen erwarteten Hörreaktionsfunktion $(fi)$

Berechnen eines $\Delta W$ für jedes Ohr des Individuums durch Subtrahieren des aus dem Basislinien-Audiogramm berechneten $W$-Werts von dem aus dem darauffolgenden Audiogramm berechneten $W$-Wert;

Erzeugen eines gewichteten Korrelationskoeffizienten, $r_W$, aus dem gemessenen Hörpegel $(Li)$ und der erwarteten Hörreaktionsfunktion $(fi)$ für jedes Ohr aus dem Basislinien-Audiogramm und dem darauffolgenden Audiogramm des Individuums gemäß einer zweiten Gleichung:

$$\mathbf{r_W} = \frac{\Sigma_i((L_{it} - L_{i0}) - (\bar{L}_t - \bar{L}_0))(f_i - \bar{f})}{\sqrt{\Sigma_i(f_i - \bar{f})^2 \Sigma_i((L_{it} - L_{i0}) - (\bar{L}_t - \bar{L}_0))^2}}$$

worin

$r_W$ eine statistische Korrelationsmetrik ist, die das Ausmaß einer linearen Beziehung zwischen einer Änderung des Hörvermögens hin zum Hörverlustzustand vom Basislinien-Audiogramm zum darauffolgenden Audiogramm im Ohr des Individuums beruhend auf der erwarteten Hörreaktionsfunktion $fi$ misst;

0 ein erstes Index ist, der für das Basislinien-Audiogramm steht;

$t$ ein zweiter Index ist, der für das darauffolgende Audiogramm steht; und

die restlichen Symbole die gleichen sind wie für die erste Gleichung definiert;

Bestimmen, ob der $\Delta W$-Wert und der entsprechende $r_W$-Wert für jedes Ohr im Individuum angibt, dass sich der Hörverlustzustand in dem Intervall zwischen dem Basislinien-Audiogramm und dem darauffolgenden Audiogramm verbessert oder verschlechtert hat oder unverändert geblieben ist; und

Schätzen einer Hörtendenz hin zum Hörverlustzustand durch Berechnen eines $\Delta W$ pro Einheitszeit in jedem Ohr des Individuums zwischen dem darauffolgenden Audiogramm und dem Basislinien-Audiogramm, ausgedrückt als absolute Änderungsrate von $W$ pro Jahr, $\Delta W$ pro Jahr und einer proportionalen Änderungsrate von $W$ pro Jahr.

2. Verfahren nach Anspruch 1, wobei der Hörverlustzustand eine frühe audiometrische Phase von Lärmschwerhörigkeit (NIHL) ist.

3. Verfahren nach Anspruch 2, wobei die $fi$-Gewichtungen eine Matrize der erwarteten Hörreaktion im Ohr für NIHL der frühen audiometrischen Stufe umfassen, die aus Matrizen ausgewählt ist, die Folgende umfassen:

0,5 kHz bei 0,0 Gewichtung, 1 kHz bei 0,0 Gewichtung, 2 kHz bei 0,0 Gewichtung, 3 kHz bei einer Gewichtung von 1,0, 4 kHz bei einer Gewichtung von 2,0, 6 kHz bei einer Gewichtung von 1,0 und 8 kHz bei einer Gewichtung von 0,5;

0,5 kHz bei 0,0 Gewichtung, 1 kHz bei 0,0 Gewichtung, 2 kHz bei 0,0 Gewichtung, 3 kHz bei einer Gewichtung von 0,5, 4 kHz bei einer Gewichtung von 2,0, 6 kHz bei einer Gewichtung von 1,0 und 8 kHz bei einer Gewichtung von 0,5; und

0,5 kHz bei 0,0 Gewichtung, 1 kHz bei 0,0 Gewichtung, 2 kHz bei 0,0 Gewichtung, 3 kHz bei einer Gewichtung von 1,0, 4 kHz bei einer Gewichtung von 2,0, 6 kHz bei einer Gewichtung von 0,5 und 8 kHz bei einer Gewichtung von 0,5.

4. Verfahren nach Anspruch 2, wobei ein $\Delta W$-Wert von zumindest 10 dBw eine Verschiebung hin zu frühem NIHL im Ohr des Individuums angibt.

5. Verfahren nach Anspruch 1, umfassend das Verwenden der gemessenen Hörpegeldaten für das Individuum aus dem Basislinien-Audiogramm und einer Vielzahl von darauffolgenden Audiogrammen, die über eine Vielzahl von aufeinanderfolgenden Zeitintervallen durchgeführt wurden.

6. Verfahren nach Anspruch 1, ferner umfassend das Umwandeln der gemessenen Hörpegeldaten aus der Vielzahl von Reintonaudiogrammen, die dem Individuum zugeordnet sind, und der erwarteten Hörreaktionsfunktion ($fi$) für den Hörverlustzustand in den gewichteten Hörpegel, der dem Individuum für jedes Ohr zugeordnet ist, um eine aggregierte Änderung über der Zeit für zumindest eines aus einer Population und einer Teilpopulation zu detektieren, wobei das Verfahren ferner folgende Schritte umfasst:

Zuweisen einer Vielzahl von Individuen zu zumindest einem aus einer definierten Population und einer definierten Teilpopulation, in der jedes Individuum eine Vielzahl von Audiogrammen aufweist, welche das Basislinien-Audiogramm, dem das Individuum beim Eintritt in die Population unterzogen wird, und zumindest das darauffolgende Audiogramm umfasst;

Anwenden eines $r_W$-Grenzkriteriumswerts auf jeden entsprechenden $\Delta W$-Wert für jedes Ohr in jedem Audiogramm unter den Individuen in der Population, um das darauffolgende Audiogramm mit dem $\Delta W$-Wert, der nicht positiv mit dem Hörverlustzustand aus den Messungen der aggregierten Populationsänderungen über der Zeit korreliert, auszuschließen;

Berechnen des Mittelwerts oder des Medianwerts der $\Delta W$/Jahr-Tendenz für jedes Ohr, einschließlich des Basislinien-Audiogramms und der darauffolgenden Audiogramme für jedes Individuum in der Population über einen Zeitraum hinweg;

Berechnen von zumindest einem aus einem arithmetischen Mittelwert und einem Medianwert der $\Delta W$/Jahr-Tendenzwerte für jedes Individuum für jedes Ohr für einen Populations-$\Delta W$/Jahr-Tendenzwert;

Berechnen des Mittelwerts oder Medianwerts des Populations-$\Delta W$/Jahr-Tendenzwerts für jedes Individuum für jedes Ohr in der Population;

Anwenden von zumindest einer statistischen Methode, um zumindest eines aus dem Messen und dem Vergleichen von Tendenzen für Hörverlustmetriken, welche den Hörverlustzustand betreffen, für zumindest eines aus der Population und der Teilpopulation durchzuführen.

7. Verfahren nach Anspruch 6, wobei der Hörverlustzustand eine frühe audiometrische Stufe von NIHL ist.

8. Verfahren nach Anspruch 7, wobei die $fi$-Gewichtungen eine Matrize der erwarteten Hörreaktion im Ohr für NIHL der frühen audiometrischen Stufe umfassen, die aus Matrizen ausgewählt ist, die Folgende umfassen:

0,5 kHz bei 0,0 Gewichtung, 1 kHz bei 0,0 Gewichtung, 2 kHz bei 0,0 Gewichtung, 3 kHz bei einer Gewichtung von 1,0, 4 kHz bei einer Gewichtung von 2,0, 6 kHz bei einer Gewichtung von 1,0 und 8 kHz bei einer Gewichtung von 0,5;

0,5 kHz bei 0,0 Gewichtung, 1 kHz bei 0,0 Gewichtung, 2 kHz bei 0,0 Gewichtung, 3 kHz bei einer Gewichtung von 0,5, 4 kHz bei einer Gewichtung von 2,0, 6 kHz bei einer Gewichtung von 1,0 und 8 kHz bei einer Gewichtung von 0,5; und

0,5 kHz bei 0,0 Gewichtung, 1 kHz bei 0,0 Gewichtung, 2 kHz bei 0,0 Gewichtung, 3 kHz bei einer Gewichtung von 1,0, 4 kHz bei einer Gewichtung von 2,0, 6 kHz bei einer Gewichtung von 0,5 und 8 kHz bei einer Gewichtung von 0,5.

9. Verfahren nach Anspruch 7, wobei die Tendenzen für Hörverlust, die den Hörverlustzustand für zumindest eines aus der Population und der Teilpopulation betreffen, angewendet werden, um die Wirksamkeit eines Programms zur Erhaltung des Hörvermögens zur Prävention von NIHL in zumindest einem aus der Population, den Populationen und den Teilpopulationen zu bewerten.

10. Verfahren nach Anspruch 7, wobei die Tendenzen für Hörverlust, die den Hörverlustzustand für zumindest eines aus der Population und der Teilpopulation betreffen, angewendet werden, um zumindest eines aus den Individuen, den Teilpopulationen, der Population und den Populationen mit erhöhtem Risiko hinsichtlich einer Entwicklung von NIHL zu identifizieren.

11. Verfahren nach Anspruch 7, wobei die Tendenzen für Hörverlust, die den Hörverlustzustand für zumindest eines aus der Population und der Teilpopulation betreffen, angewendet werden, um die Wirksamkeit eines Präventionsverfahrens zur Kontrolle von Lärmexposition innerhalb von zumindest einem aus der Population, den Populationen und den Teilpopulationen zu messen.

**EP 4 255 297 B1**

**12.** System, das zumindest einen Prozessor und ein nichtflüchtiges computerlesbares Medium umfasst, welches Befehle umfasst, die, wenn sie von dem zumindest einen Prozessor ausgeführt werden, bewirken, dass das System ein Verfahren nach einem der Ansprüche 1 bis 11 durchführt.

**13.** Nichtflüchtiges computerlesbares Medium, welches Befehle umfasst, die, wenn sie von zumindest einem Prozessor ausgeführt werden, bewirken, dass der zumindest eine Prozessor ein Verfahren nach einem der Ansprüche 1 bis 11 durchführt.

**Revendications**

**1.** Procédé de détection d'un changement dans un état de perte auditive, comprenant les étapes comprenant le fait de :

identifier l'état de perte auditive mesuré dans au moins un audiogramme tonal par un motif caractéristique et une amplitude relative dans des niveaux auditifs mesurés dans une oreille dans au moins l'une parmi une fréquence sonore, une gamme de fréquences, ou dans une combinaison de fréquences, en comparaison avec un ou plusieurs niveaux auditifs mesurés à une pluralité d'autres fréquences dans l'audiogramme ;
fournir une fonction de réponse auditive attendue, $f_i$, pour l'état de perte auditive calculé à partir d'une pluralité de fréquences sonores, i, mesurées dans l'audiogramme de l'oreille, où $f_i$ est un produit scalaire, qui comprend une valeur proportionnelle sans unité, attribuée au niveau auditif ;
générer un niveau auditif pondéré, W, à partir d'un niveau auditif mesuré associé au sujet pour chaque oreille à partir de l'audiogramme et de la fonction de réponse auditive attendue ($f_i$) pour l'état de perte auditive selon une première équation :

$$\mathbf{W} = \bar{L} + \frac{\Sigma_i L_i(f_i - \bar{f})}{\Sigma_i (f_i - \bar{f})^2}(f_m - \bar{f})$$

dans laquelle

$W$ est le niveau auditif pondéré qui synthétise la correspondance entre l'audiogramme mesuré et l'état de perte auditive dans l'oreille, exprimé sous la forme d'une valeur numérique en unités équivalentes de décibels, dB, appelées « dBw » ;
$i$ sont les fréquences sonores pour lesquelles une réponse auditive est attendue et un niveau auditif a été mesuré dans l'audiogramme de l'oreille ;
$L_i$ est le niveau auditif mesuré pour chaque fréquence sonore ($i$) dans l'oreille, telle qu'enregistrée dans l'audiogramme associé au sujet, mesuré en dB ;
$\bar{L}$ est une première moyenne arithmétique des niveaux auditifs mesurés $L_i$, calculée en dB ;
$f_i$ est la fonction de réponse auditive attendue à chaque fréquence sonore enregistrée ($i$) ;
$\bar{f}$ est une deuxième moyenne arithmétique des valeurs $f_i$ ;
$\Sigma_i$ représente une sommation sur les valeurs i ; et
$f_m$ est la valeur maximale dans l'ensemble des valeurs $f_i$ ;
calculer W pour un audiogramme de référence et calculer W pour un audiogramme subséquent pour chaque oreille du sujet, en utilisant la même fonction de réponse auditive attendue ($f_i$) ;
calculer une valeur ΔW pour chaque oreille du sujet en soustrayant la valeur W calculée à partir de l'audiogramme de référence de la valeur W calculée à partir de l'audiogramme subséquent ;
générer un coefficient de corrélation pondéré, $r_w$, à partir du niveau auditif mesuré ($L_i$) et de la fonction de réponse auditive attendue ($f_i$) pour chaque oreille à partir de l'audiogramme de référence et de l'audiogramme subséquent du sujet, selon une deuxième équation :

$$\mathbf{r_w} = \frac{\Sigma_i((L_{it} - L_{i0}) - (\bar{L}_t - \bar{L}_0))(f_i - \bar{f})}{\sqrt{\Sigma_i(f_i - \bar{f})^2 \Sigma_i((L_{it} - L_{i0}) - (\bar{L}_t - \bar{L}_0))^2}}$$

où

$r_w$ est une métrique de corrélation statistique qui mesure l'étendue d'une relation linéaire entre une variation dans l'audition vers l'état de perte auditive, de l'audiogramme de référence à l'audiogramme subséquent dans l'oreille du sujet, sur la base de la fonction de réponse auditive attendue ($f_i$) ;

0 est un premier indice qui désigne l'audiogramme de référence ;

*t* est un deuxième indice qui désigne l'audiogramme subséquent ; et

les autres symboles sont les mêmes que ceux définis dans la première équation ;

déterminer si la valeur $\Delta$W et la valeur $r_w$ correspondante pour chaque oreille du sujet indiquent que l'état de perte auditive s'est amélioré, aggravé ou est resté inchangé au cours de l'intervalle de temps entre l'audiogramme de référence et l'audiogramme subséquent ; et

estimer une tendance de l'audition vers l'état de perte auditive en calculant une valeur $\Delta$W par unité de temps dans chaque oreille du sujet entre l'audiogramme subséquent et l'audiogramme de référence, exprimée sous la forme d'un taux de variation absolu en W par an, d'une $\Delta$W/an, et d'un taux de variation proportionnel en W par an.

2. Procédé selon la revendication 1, dans lequel l'état de perte auditive est une phase audiométrique précoce de perte auditive induite par le bruit (NIHL).

3. Procédé selon la revendication 2, dans lequel les pondérations $f_i$ comprennent un modèle de la réponse auditive attendue dans l'oreille pour une phase audiométrique précoce de NIHL, choisi parmi les modèles constitués par :

0,5 kHz avec une pondération de 0,0, 1 kHz avec une pondération de 0,0, 2 kHz avec une pondération de 0,0, 3 kHz avec une pondération de 1,0, 4 kHz avec une pondération de 2,0, 6 kHz avec une pondération de 1,0 et 8 kHz avec une pondération de 0,5 ;

0,5 kHz avec une pondération de 0,0, 1 kHz avec une pondération de 0,0, 2 kHz avec une pondération de 0,0, 3 kHz avec une pondération de 0,5, 4 kHz avec une pondération de 2,0, 6 kHz avec une pondération de 1,0 et 8 kHz avec une pondération de 0,5 ; et

0,5 kHz avec une pondération de 0,0, 1 kHz avec une pondération de 0,0, 2 kHz avec une pondération de 0,0, 3 kHz avec une pondération de 1,0, 4 kHz avec une pondération de 2,0, 6 kHz avec une pondération de 0,5 et 8 kHz avec une pondération de 0,5.

4. Procédé selon la revendication 2, dans lequel la valeur $\Delta$W d'au moins 10 dBw indique un décalage vers une NIHL précoce dans l'oreille du sujet.

5. Procédé selon la revendication 1, comprenant le fait d'utiliser des données de niveau auditif mesuré pour le sujet, provenant de l'audiogramme de référence et d'une pluralité d'audiogrammes subséquents réalisés sur une pluralité d'intervalles de temps subséquents.

6. Procédé selon la revendication 1, comprenant en outre le fait de transformer les données de niveau auditif mesuré provenant de la pluralité d'audiogrammes tonals associés au sujet et de la fonction de réponse auditive attendue ($f_i$) pour l'état de perte auditive en le niveau auditif pondéré associé au sujet pour chaque oreille, afin de détecter une variation globale dans le temps pour au moins l'une parmi une population et une sous-population, le procédé comprenant en outre les étapes comprenant le fait de :

disposer d'une pluralité de sujets affectés à au moins l'une parmi une population définie et une sous-population définie, dans laquelle chaque sujet a une pluralité d'audiogrammes qui comprend l'audiogramme de référence qui est administré au sujet lors de son entrée dans la population, et au moins l'audiogramme subséquent ;

appliquer une valeur de critère de coupure $r_w$ à chaque valeur $\Delta$W correspondante pour chaque oreille dans chaque audiogramme parmi les sujets de la population, afin d'exclure l'audiogramme subséquent, dont la valeur $\Delta$W n'est pas en corrélation positive avec l'état de perte auditive, des mesures des variations globales de la population au fil du temps ;

calculer la valeur moyenne ou médiane de la tendance $\Delta$W/an pour chaque oreille, incluant l'audiogramme de référence et les audiogrammes subséquents, pour chaque sujet de la population sur une période de temps ;

calculer au moins l'une parmi une moyenne arithmétique ou une médiane des valeurs de tendance $\Delta$W/an pour chaque sujet pour chaque oreille, afin d'obtenir une valeur de tendance $\Delta$W/an de population ;

calculer la valeur moyenne ou médiane de la valeur de tendance $\Delta$W/an de population pour chaque sujet pour chaque oreille dans la population ;

appliquer au moins une méthode statistique pour effectuer au moins l'une des opérations consistant à mesurer et à comparer des tendances pour des métriques de perte auditive qui se rapportent à l'état de perte auditive pour au moins l'une parmi la population et la sous-population.

**7.** Procédé selon la revendication 6, dans lequel l'état de perte auditive est une phase audiométrique précoce de NIHL.

**8.** Procédé selon la revendication 7, dans lequel les pondérations $f_i$ comprennent un modèle de la réponse auditive attendue dans l'oreille pour une phase audiométrique précoce de NIHL, choisi parmi les modèles constitués par :

0,5 kHz avec une pondération de 0,0, 1 kHz avec une pondération de 0,0, 2 kHz avec une pondération de 0,0, 3 kHz avec une pondération de 1,0, 4 kHz avec une pondération de 2,0, 6 kHz avec une pondération de 1,0 et 8 kHz avec une pondération de 0,5 ;
0,5 kHz avec une pondération de 0,0, 1 kHz avec une pondération de 0,0, 2 kHz avec une pondération de 0,0, 3 kHz avec une pondération de 0,5, 4 kHz avec une pondération de 2,0, 6 kHz avec une pondération de 1,0 et 8 kHz avec une pondération de 0,5 ; et
0,5 kHz avec une pondération de 0,0, 1 kHz avec une pondération de 0,0, 2 kHz avec une pondération de 0,0, 3 kHz avec une pondération de 1,0, 4 kHz avec une pondération de 2,0, 6 kHz avec une pondération de 0,5 et 8 kHz avec une pondération de 0,5.

**9.** Procédé selon la revendication 7, dans lequel les tendances de perte auditive qui se rapportent à l'état de perte auditive pour au moins l'une parmi la population et la sous-population sont appliquées pour évaluer l'efficacité d'un programme de conservation d'audition pour prévenir la perte auditive induite par le bruit, NIHL, dans au moins l'une parmi la population, les populations et les sous-populations.

**10.** Procédé selon la revendication 7, dans lequel les tendances de perte auditive qui se rapportent à l'état de perte auditive pour au moins l'une parmi la population et la sous-population sont appliquées pour identifier au moins l'un parmi les sujets, les sous-populations, la population et les populations présentant un risque accru de développer une perte auditive induite par le bruit, NIHL.

**11.** Procédé selon la revendication 7, dans lequel les tendances de perte auditive qui se rapportent à l'état de perte auditive pour au moins l'un parmi la population et la sous-population sont appliquées pour mesurer l'efficacité d'une méthode préventive de contrôle de l'exposition au bruit au sein d'au moins l'une parmi la population, les populations et les sous-populations.

**12.** Système comprenant au moins un processeur et un support non transitoire lisible par ordinateur comprenant des instructions qui, lorsqu'elles sont exécutées par l'au moins un processeur, amènent le système à mettre en œuvre le procédé selon l'une quelconque des revendications 1 à 11.

**13.** Support non transitoire lisible par ordinateur comprenant des instructions qui, lorsqu'elles sont exécutées par au moins un processeur, amènent l'au moins un processeur à mettre en œuvre le procédé selon l'une quelconque des revendications 1 à 11.

**X = Left Ear   O = Right Ear**

**FIG. 1**

**FIG. 2**

**FIG. 3A**

**FIG. 3B**

FIG. 4

FIG. 5

EP 4 255 297 B1

FIG. 6

**FIG. 7**

FIG. 8

**FIG. 9**

FIG. 10

FIG. 11

FIG. 12

FIG. 13

**FIG. 14**

**FIG. 15**

**A**

Scatter plot of delta dBw vs. Correlation with early NIHL, LEFT Ear, All Periodic Tests

**B**

Scatter plot of delta dBw vs. Correlation with early NIHL, RIGHT Ear, All Periodic Tests

**FIG. 16**

**EP 4 255 297 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 63122083

- US 2017300631 A

**Non-patent literature cited in the description**

- Medical Surveillance. **CRANER, J.** Current Occupational and Environmental Medicine. McGraw Hill Companies, Inc, 2014